# EUROPEAN PATENT APPLICATION

(11) **EP 4 215 228 A1**
(43) Date of publication of application: **26.07.2023**
(21) Application number: 23151337.5
(22) Date of filing: 12.01.2023
(51) Int. Cl.: A61M 5/142, A61M 5/145, A61M 5/172, A61B 5/145

(54) **AMBULATORY INFUSION PUMP DEVICE WITH INTEGRATED SENSOR**

(30) Priority: 21.01.2022 US 202263267022 P; 31.05.2022 US 202263365544 P; 26.10.2022 US 202218049769
(71) Applicant: Medtronic MiniMed, Inc., Northridge, CA 91325 (US)
(72) Inventor: Smith, Roger E., Northridge, 91325 (US)
(74) Representative: Zimmermann & Partner Patentanwälte mbB

(57) **Abstract**

Integrated sensor and infusion devices are disclosed herein. The present technology includes, for example, an integrated sensor and infusion device for sensing physiological parameter(s) and delivering a medicament to a body of a user based at least in part on the sensed parameter(s). The device can comprise an insertion assembly comprising a carrier assembly comprising a cannula carrier, a trocar assembly removably coupled to the carrier assembly, and a drive assembly comprising a torsion spring coupled to the trocar assembly such that, when actuated, the torsion spring rotates to drive the trocar assembly and the carrier assembly axially downward to insert an infusion cannula and sensor electrode into a user's skin. The drive assembly can comprise a plurality of coupled drive wheels and/or a scissor assembly with multiple interacting links.

## Description

### CROSS-REFERENCE TO RELATED APPLICATIONS

This application claims the benefit of priority to U.S. Provisional Application No. 63/267,022, filed January 21, 2022, and U.S. Provisional Application No. 63/365,544, filed May 31, 2022, which are incorporated herein by reference in their entirety.

### TECHNICAL FIELD

The present technology relates generally to medical devices, and more particularly, to insulin delivery devices with integrated continuous glucose monitors.

### BACKGROUND

Ambulatory infusion pumps are relatively small, at least substantially self-contained devices that are used to introduce drugs and other infusible substances (collectively "medicament") into users' bodies. Some infusion pumps are configured to be worn on a belt, carried in a clothing pocket, or the like. Other infusion pumps are configured to be adhered to skin in patch-like fashion. Infusion pumps are advantageous in that they may be used to, for example, subcutaneously introduce (or "infuse") medicament on an ongoing or even continuous basis outside of a clinical environment. Infusion pumps are also advantageous in that they greatly reduce the frequency of subcutaneous access events such as needle-based shots. One example of a medicament that may be introduced by an infusion pump is a liquid formulation of insulin. Other exemplary medicaments that may be introduced by an infusion pump include, but are not limited to, drugs that treat cancers and drugs that suppress the perception of pain.

In some instances, ambulatory infusion pumps may automatically dispense medicament (e.g., insulin) based on measurements obtained by a real-time monitoring device. In the case of an insulin infusion pump, such a real-time monitoring device can take the form of a continuous glucose monitor (CGM). Examples of such CGMs include wearable devices having a sensor component that can be inserted into the user's skin to record a user's glucose levels continuously or periodically over time.

The use of an infusion pump device and a separate sensor device creates complexity for the user and requires significant space on a user's skin for implementation. Although combination devices have been proposed that integrate both an infusion pump and a CGM, such devices are generally bulky and expensive. Additionally, in combination devices the sensor component may be positioned too near to the insulin delivery cannula to obtain accurate physiological insulin readings of the user. Accordingly, there remains a need to develop improved ambulatory infusion pump devices with integrated sensors.

### SUMMARY

In one aspect, the present technology includes an integrated sensor and infusion device. The device includes a reservoir assembly including a reservoir configured to retain medicament therein. The device also includes a sensor electronics assembly configured to receive signals from a sensor. The device also includes an insertion assembly having a carrier assembly including a cannula carrier, an infusion cannula extending downwardly away from the cannula carrier, and a sensor electrode extending downwardly away from the cannula carrier at a position laterally spaced apart from the infusion cannula, where the infusion cannula is fluidically coupled to the reservoir, a trocar assembly including a trocar link, a first trocar configured to removably engage the infusion cannula, and a second trocar configured to removably engage the sensor electrode, the trocar assembly removably coupled to the carrier assembly, and a drive assembly includes a torsion spring coupled to the trocar assembly such that, when actuated, the torsion spring rotates to drive the trocar assembly and the carrier assembly axially downward to insert the first trocar, the infusion cannula, the second trocar, and the sensor electrode into a user's skin.

The device may also include a device housing in which each of the components of the device are configured to be housed prior to insertion of the first trocar, the infusion cannula, the second trocar, and the sensor electrode into the user's skin, and where the first trocar, the infusion cannula, the second trocar, and the sensor electrode extend out of the housing for insertion into the user's skin. The device may also be configured such that a first amount of rotation by the torsion spring drives the first trocar, the infusion cannula, the second trocar, and the sensor electrode into the user's skin, and where a second amount of rotation retracts the first trocar and the second trocar from the user's skin while leaving the infusion cannula and the sensor electrode in the user's skin. The torsion spring is actuated via a manual trigger mechanism, and/or may be actuated via a remote trigger mechanism. The first trocar can be configured to extend within a lumen of the infusion cannula. The sensor electrode can be configured to be removably received within a recess of the second trocar.

In some embodiments, the drive assembly further includes a scissor assembly coupled to the torsion spring via a drive wheel, the scissor assembly includes a first link coupled to the trocar link at a first region and a second link coupled to the trocar link at a second region, where the torsion spring, when actuated, is configured to rotate the drive wheel to cause, via the scissor assembly, the trocar link to move axially to drive the first trocar, the infusion cannula, the second trocar, and the sensor electrode into the user's skin. In some embodiments, the drive wheel is disposed within a housing and the drive wheel includes a pin received within a cam slot of the first link, such that rotation of the drive wheel causes the pin to slide within the cam slot and causes the first link to rotate relative to the drive wheel housing. The second link be coupled to the first link such that rotation of the first link causes opposite rotation of the second link. The drive assembly, when actuated, may move the scissor assembly from an unfired position in which the infusion cannula and sensor electrode are disposed within a housing of the device to an inserted position in which the infusion cannula and sensor electrode extend beyond the housing of the device. In some embodiments, in the unfired position, the first link and second link assume an expanded state in which they extend along non-parallel axes, and where, in the inserted position, the first link and the second link assume a collapsed state in which they extend more nearly parallel to one another.

In some embodiments, the drive assembly further includes a first drive wheel coupled to the torsion spring and a second drive wheel mated with the first drive wheel such that rotation of the first drive wheel causes rotation of the second drive wheel. The first drive wheel can include a first pin and the second drive wheel can include a second pin, each of the first pin and the second pin extending into a cam slot of the trocar link such that rotation of the first drive wheel and the second drive wheel causes the trocar link to move axially. Axial movement of the trocar link in the downward direction may cause axial movement of the carrier assembly in the downward direction. Rotation of the first drive wheel in a first direction may cause rotation of the second drive wheel in a second, opposite direction. The device may also include where the cannula carrier includes a first cam track configured to releasably receive the first pin therein and a second cam track configured to releasably receive the second pin therein. The device may also be configured such that the first and second pins engage the first and second cam tracks, respectively, when the trocar assembly and the carrier assembly are each in a downwardly inserted position, and where the first and second pins disengage from the first and second cam tracks when the trocar assembly is retracted upward with respect to the carrier assembly.

In another aspect, an integrated sensor and infusion device includes a torsion spring, a drive wheel coupled to the torsion spring, a scissor assembly coupled to the drive wheel, the scissor assembly includes a first link and a second link, a slide having first and second trocars coupled thereto, the slide coupled to the first link at a first region and coupled to the second link at a second region, an infusion cannula removably coupled to the first trocar, and a sensor electrode removably coupled to the second trocar, where the torsion spring, when actuated, is configured to rotate the drive wheel to cause, via the scissor assembly, the slide to move axially to drive the first trocar, the infusion cannula, the second trocar, and the sensor electrode into a user's skin.

In some embodiments, each of the components of the device are configured to be housed within a device housing prior to insertion of the first trocar, the infusion cannula, the second trocar, and the sensor electrode into the user's skin, and where the first trocar, the infusion cannula, the second trocar, and the sensor electrode extend out of the housing for insertion into the user's skin. In some embodiments, a first amount of rotation by the torsion spring drives the first trocar, the infusion cannula, the second trocar, and the sensor electrode into the user's skin, and where a second amount of rotation retracts the first trocar and the second trocar from the user's skin while leaving the infusion cannula and the sensor electrode in the user's skin. In some embodiments, the torsion spring is actuated via a manual trigger mechanism. In some embodiments, the torsion spring is actuated via a remote trigger mechanism. In some embodiments, the first trocar is configured to extend within a lumen of the infusion cannula. In some embodiments, the sensor electrode is configured to be removably received within a recess of the second trocar.

In some embodiments, the drive wheel is disposed within a drive wheel housing and the drive wheel includes a pin received within a cam slot of the first link, such that rotation of the drive wheel causes the pin to slide within the cam slot and causes the first link to rotate relative to the drive wheel housing. In some embodiments, the second link is coupled to the first link such that rotation of the first link causes opposite rotation of the second link. In some embodiments, the drive assembly, when actuated, moves the scissor assembly from an unfired position in which the infusion cannula and sensor electrode are disposed within a housing of the device to an inserted position in which the infusion cannula and sensor electrode extend beyond the housing of the device. In some embodiments, in the unfired position, the first link and second link assume an expanded state in which they extend along non-parallel axes, and where, in the inserted position, the first link and the second link assume a collapsed state in which they extend parallel to one another.

In another aspect, an integrated sensor and infusion device includes a torsion spring, a first drive wheel coupled to the torsion spring, a second drive wheel mated with the first drive wheel such that rotation of the first drive wheel causes rotation of the second drive wheel, a slide having first and second trocars coupled thereto, the slide coupled to the first drive wheel at a first region and coupled to the second drive wheel at a second region, an infusion cannula removably coupled to the first trocar, and a sensor electrode removably coupled to the second trocar, where the torsion spring, when actuated, is configured to rotate the drive wheel to cause the slide to move axially to drive the first trocar, the infusion cannula, the second trocar, and the sensor electrode into a user's skin.

In some embodiments, each of the components of the device are configured to be housed within a device housing prior to insertion of the first trocar, the infusion cannula, the second trocar, and the sensor electrode into the user's skin, and where the first trocar, the infusion cannula, the second trocar, and the sensor electrode extend out of the housing for insertion into the user's skin. In some embodiments, a first amount of rotation by the torsion spring drives the first trocar, the infusion cannula, the second trocar, and the sensor electrode into the user's skin, and where a second amount of rotation retracts the first trocar and the second trocar from the user's skin while leaving the infusion cannula and the sensor electrode in the user's skin. In some embodiments, the first amount of rotation is approximately 180 degrees, and the second amount of rotation is an additional approximately 180 degrees. In some embodiments, the torsion spring is actuated via a manual trigger mechanism. In some embodiments, the torsion spring is actuated via a remote trigger mechanism. In some embodiments, the first trocar is configured to extend within a lumen of the infusion cannula. In some embodiments, the sensor electrode is configured to be removably received within a recess of the second trocar. In some embodiments, the first drive wheel includes a first pin, and the second drive wheel includes a second pin, each of the first pin and the second pin extending into a cam slot of the trocar link such that rotation of the first drive wheel and the second drive wheel causes the trocar link to move axially. In some embodiments, axial movement of the trocar link in the downward direction causes axial movement of the carrier assembly in the downward direction. In some embodiments, rotation of the first drive wheel in a first direction causes rotation of the second drive wheel in a second, opposite direction. In some embodiments, the cannula carrier includes a first cam track configured to releasably receive the first pin therein and a second cam track configured to releasably receive the second pin therein. In some embodiments, the trocar assembly and the carrier assembly are each in a downwardly inserted position, and where the first and second pins disengage from the first and second cam tracks when the trocar assembly is retracted upward with respect to the carrier assembly.

In some embodiments, the reservoir contains medicament comprising insulin. In some embodiments, the sensor electrode includes a glucose monitor electrode. In some embodiments, the infusion cannula extends at least 3, 4, 5, 6, 7, 8, 9, 10 mm or more into the user's skin. In some embodiments, the sensor electrode extends at least 7, 8, 9, 10, 11, 12, 13, 14 mm or more into the user's skin. In some embodiments, the infusion cannula and the sensor electrode are laterally spaced apart from one another by at least 10, 11, 12, 13, 14, 15, 16, 17, 18, 19, 20 mm or more. In some embodiments, when positioned over the user's skin, a greatest height of the device over the user's skin is less than about 10, 11, 12, 13, 14, 15, 16, 17, 18, 19, 20 mm or more.

In another aspect, a method for operating an integrated sensor and infusion device, the method includes disposing a device housing over a user's skin, the device includes a drive assembly including a torsion spring, an infusion cannula fluidically coupled to a reservoir configured to hold medicament therein, and a sensor electrode. The method also includes actuating the drive assembly to rotate the torsion spring, thereby driving the infusion cannula and the sensor electrode into a user's skin. The method also includes obtaining physiological measurement(s) via the sensor electrode. The method also includes, based at least in part on the physiological measurement(s), delivering medicament into the user's body via the infusion cannula.

In some embodiments, the medicament includes insulin. In some embodiments, the physiological measurement(s) includes a blood glucose measurement. In some embodiments, the device further includes a cannula carrier coupled to the infusion cannula and the sensor electrode. In some embodiments, the device further includes a trocar assembly including a trocar link coupled to a first trocar configured to releasably engage the infusion cannula and a second trocar configured to releasably engage the sensor electrode. In some embodiments, the drive assembly further includes a scissor assembly coupled to the torsion spring via a drive wheel, and where rotation of the drive wheel causes the scissor assembly to move from an expanded configuration in which first and second links of the scissor assembly are oriented along intersecting axes to a collapsed configuration in which the first and second links of the scissor assembly are oriented substantially parallel to one another. In some embodiments, the drive assembly further includes a first drive wheel coupled to the torsion spring and a second drive wheel mated with the first drive wheel such that rotation of the first drive wheel causes rotation of the second drive wheel, and where rotation of the first drive wheel and the second drive wheel drives the infusion cannula and the sensor electrode into a user's skin.

Other technical features may be readily apparent to one skilled in the art from the following figures, descriptions, and claims.

### BRIEF DESCRIPTION OF THE DRAWINGS

Many aspects of the present disclosure can be better understood with reference to the following drawings. The components in the drawings are not necessarily to scale. Instead, emphasis is placed on clearly illustrating the principles of the present disclosure.
FIG. 1A is a perspective view of an infusion device with an integrated sensor that includes a disposable assembly and a durable assembly in accordance with several embodiments of the present technology.
FIG. 1B is a perspective view of the device of FIG. 1A with the durable assembly removed.
FIG. 2 is a perspective view of a disposable assembly of the FIG. 1B with the upper housing omitted for clarity.
FIGS. 3A and 3B illustrate perspective views of an insertion assembly in an unfired and fired position, respectively, in accordance with several embodiments of the present technology.
FIGS. 4A and 4B illustrate enlarged perspective detail views of the insertion assembly in an unfired and fired position, respectively.
FIG. 5 is an exploded perspective view of the drive assembly of the insertion assembly shown in FIGS. 4A and 4B.
FIG. 6 illustrates a perspective view of a trocar slider of the drive assembly in accordance with several embodiments of the present technology.
FIGS. 7A-7C illustrate front perspective, rear perspective, and cross-sectional views, respectively, of a cannula carrier assembly in accordance with several embodiments of the present technology.
FIGS. 8A and 8B illustrate front and rear perspective views, respectively, of a sensor assembly in accordance with several embodiments of the present technology.
FIGS. 9A and 9B illustrate perspective views of a scissor assembly engaged with a crank shaft of the drive assembly in accordance with several embodiments of the present technology.
FIGS. 10A-10C illustrate perspective views of the links of the scissor assembly in accordance with several embodiments of the present technology
FIGS. 11A and 11B illustrate perspective views of a drive assembly in an unfired and inserted position, respectively, in accordance with several embodiments of the present technology.
FIG. 12A is a perspective view of an infusion device with an integrated sensor that includes a disposable assembly and a durable assembly in accordance with several embodiments of the present technology.
FIG. 12B is a perspective view of the device of FIG. 12A with the durable assembly removed.
FIG. 12C is a perspective view of a disposable assembly of the FIG. 12B with the upper housing omitted for clarity.
FIG. 13 is a perspective view of an insertion assembly in accordance with several embodiments of the present technology.
FIG. 14 is a perspective view of a drive assembly of the insertion assembly in accordance with several embodiments of the present technology.
FIG. 15 is a perspective view of a trocar assembly in accordance with several embodiments of the present technology.
FIGS. 16A and 16B are perspective front and rear views, respectively, of a cannula carrier assembly with a sensor assembly in accordance with several embodiments of the present technology.
FIG. 17 is a perspective view of the sensor assembly shown in FIGS. 16A and 16B in accordance with several embodiments of the present technology.
FIGS. 18A and 18B are end views of the insertion assembly in an unfired and inserted position, respectively, in accordance with several embodiments of the present technology.
FIGS. 19A-19C illustrate several perspective views of a disposable assembly of a multi-sensor device in accordance with several embodiments of the present technology.

### DETAILED DESCRIPTION

### I. Overview

The present technology relates to wearable devices that can both deliver medicament (e.g., via infusion using a cannula inserted into a user's skin) and also monitor one or more physiological parameters (e.g., via a sensor electrode or other sensing element inserted into the user's skin). In some embodiments, the medicament delivered can include insulin, and the sensing electrode can be configured to obtain glucose measurements used to derive or infer the user's blood glucose levels. According to some examples, the device can include a housing that contains the components therein, including a reservoir, a motor to deliver medicament, and an insertion assembly to drive an infusion cannula and sensor electrode out of the housing and into the user's skin. In operation, the housing can be placed over the user's skin. After applying the device to the skin, the user activates the insertion assembly to insert the infusion cannula and the sensor electrode subcutaneously. While it is generally desirable to minimize the size of the device, there are practical limits to size reduction. For example, placing the sensor electrode and the infusion cannula too close together can reduce the accuracy of readings obtained by the sensor. As such, there may also be a minimum lateral separation between the infusion cannula and the sensor electrode. Additionally, to effectively deliver medicament and to obtain accurate sensor readings, the infusion cannula and/or the sensor electrode should be inserted to a sufficient depth beneath the surface of the user's skin. Accordingly, there may be a minimum "travel" distance required by the insertion mechanism to enable the infusion cannula and sensor electrode to penetrate the user's skin to a sufficient depth. Achieving these aspects (sufficient lateral separation and sufficient travel depth) can often lead to bulky and/or undesirably large devices.

In embodiments of the present technology, particular insertion assemblies can be used that achieve both sufficient travel depth and sufficient lateral separation without requiring undue device size (e.g., without unduly increasing the height of the device). In various examples, the drive assembly can take the form of torsion spring coupled to a trocar assembly that includes first and second trocars configured to releasably engage with an infusion cannula and a sensor electrode, respectively. When the drive assembly is actuated, the torsion spring rotates to cause the trocar assembly to fire axially downwardly, thereby driving both the infusion cannula and the sensor electrode axially downwardly and out of the device housing. By continued rotation of the torsion spring, the trocar assembly can be retracted upwardly into the device housing while the infusion cannula and sensor electrode remain in place inserted into the user's skin.

In some embodiments, the drive assembly can take the form of a scissor assembly coupled to the torsion spring. The scissor assembly can include first and second links that are aligned along intersecting axes (e.g., forming an X-shape) in an expanded, unfired state. When the torsion spring rotates to fire the trocar assembly, the first and second links move to a collapsed state in which they are more closely parallel to one another. In this process, the trocar assembly moves axially downwardly, thereby also causing the infusion cannula and sensor electrode to be moved axially downwardly and into the user's skin.

In some embodiments, the drive assembly can take the form of a dual-crank assembly in which a first drive wheel is coupled to the torsion spring, and a second drive wheel is coupled to the first drive wheel such that rotation of the torsion spring causes the first and second drive wheels to rotate in opposite directions. Each of the first and second drive wheels can include a respective pin configured to engage a cam slot of the trocar assembly, such that rotation of the drive wheels causes axial movement of the trocar assembly (e.g., in the downward direction to fire the infusion cannula and sensor electrode beyond the device housing, and in the upward direction to retract the trocar assembly back into the device housing).

### II. Exemplary Assemblies of Devices with Scissor Drive Assemblies

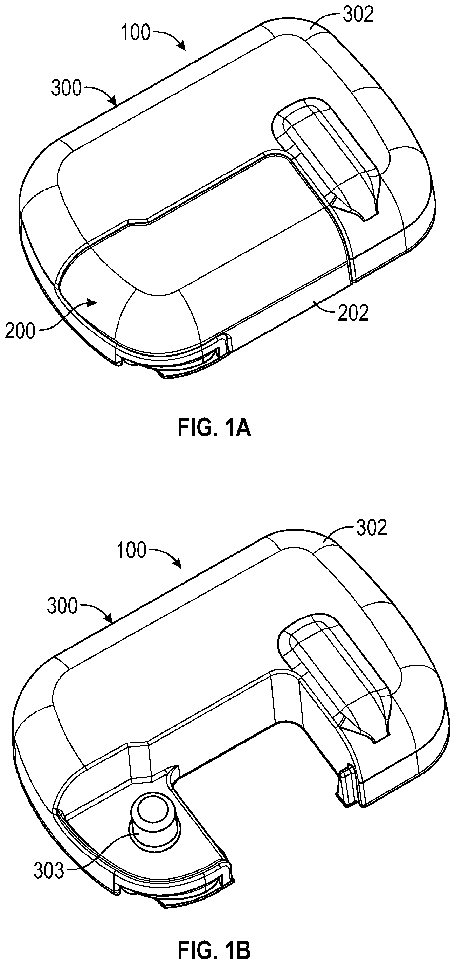

FIG. 1A is a perspective view of an infusion device with an integrated sensor ("device 100") that includes a durable assembly 200 and a disposable assembly 300 in accordance with several embodiments of the present technology. FIG. 1B illustrates the device 100 with the durable assembly 200 removed. In operation, a bottom side of the device (not shown) is configured to be adhered to the user's skin with the top side facing away from the user's skin. The device 100 includes the durable assembly 200 and the disposable assembly 300, each having respective housings 202 and 302. The durable assembly 200 and disposable assembly 300 can be disposed on an adhesive pad (not shown) with adhesive backing for securing to the user's skin. In various embodiments, the device 100 can have a length of about 35-60 mm; a width of about 30-45 mm; and an overall thickness or height of about 8-18 mm. Suitable housing materials include, but are not limited to, plastic or other materials having a modulus of elasticity of 0.2-1.0 million psi.

To use the infusion device 100, the user (e.g., the patient) connects the disposable assembly 300 to the durable assembly 200. Unless the reservoir of the disposable assembly 300 has been sufficiently pre-loaded, the user injects a desired amount of medicament into the reservoir via a fill port disposed on a lower surface of the device 100. To adhere the device 100 to the user, an adhesive backing may be exposed on the bottom side of the device 100 and the device 100 can be applied to the skin surface. In some embodiments, the user then triggers insertion which causes both an infusion cannula and a sensor electrode to be inserted beyond the housing 302 and into the user's skin, as described in more detail below.

The durable assembly 200 may include a housing 202, which encompasses one or more electronic components, for example a buzzer or other alarm device, one or more batteries or other energy supply, a microprocessor, a coil assembly (which functions as a motor stator) including one or more Hall-effect sensors, one or more wireless transceivers, and/or any other suitable components configured to power and/or control operation of the device 100. In some embodiments, the energy supply is a rechargeable battery, such as a rechargeable lithium-ion battery, with enough power to drive the motor continuously without needing a capacitor or other additional energy storage device.

Referring specifically to FIG. 1B, the durable assembly 200 can include a receptable (not shown) configured to fit over protrusion 303 of the disposable assembly 300 such that the two are releasably mated together. The electronic components within the durable assembly 200 can wirelessly communicate with corresponding components within the disposable assembly 300. For example, a magnetic stator within the durable assembly 200 can drive a magnetic motor rotor 311 motor within the disposable assembly 300, which in turn can drive a pusher 309 coupled to a plunger within a reservoir of the disposable assembly 300, e.g., using electromagnetic torque coupling, which is a coupling without direct mechanical coupling or electrical contact. These designs afford the additional advantage of being relatively simple to make waterproof, or at least water resistant.

Moreover, in some embodiments the electronic components within the disposable assembly 300 can transmit data to electronic components within the durable assembly 200. For example, a continuous glucose monitor (CGM) within the disposable assembly 300 can collect low-level (e.g., millivolt) CGM signals that are conditioned (e.g., amplified, processed, digitized, etc.) within electronics of the disposable assembly 300. This pre-processed CGM data may then be wirelessly transmitted to electronic components within the durable assembly 200 for additional processing and/or for wireless transmission to external devices (e.g., smartphone, tablet, etc.). Such transmission can include, for example, Bluetooth Low Energy (BLE) or other power-efficient methods for wirelessly transmitting the data. Because the disposable assembly 300 and the durable assembly 200 are positioned so closely together, and because the transmission path is consistent and well-defined, the power required for transmission can be relatively low (as compared to systems in which a discrete CGM is positioned on an opposite side of a patient's abdomen from a patch pump device). Additionally, by providing a power source (e.g., a battery) within the disposable assembly 300, this pre-processing and data communication between the durable assembly 200 and the disposable assembly 300 can be carried out with no physical electrical connections between the two.

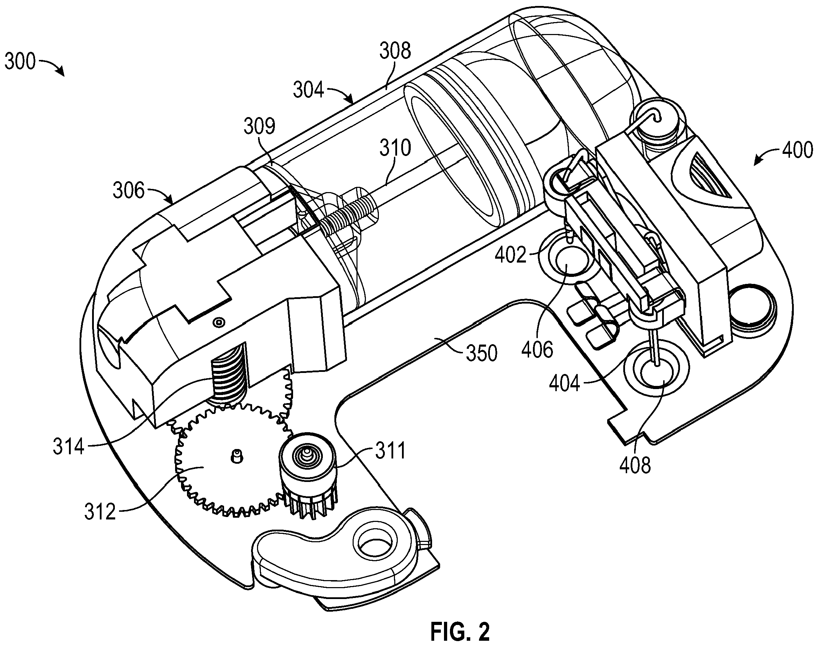

The disposable assembly 300, shown in more detail in FIG. 2 with a portion of the housing 302 removed, may include a reservoir assembly 304, and an insertion assembly 400, each mounted on a baseplate 350. The reservoir assembly 304 can comprise a drive assembly 306, a reservoir 308, and a pusher 309 coupled to a plunger 310. The pusher 309 can be coupled to the drive assembly, and both the pusher 309 and the plunger 310 can be contained within the reservoir 308. The drive assembly 306 can comprise a magnetic motor rotor 311 and a gear train 312. The gear train 312 is coupled to the pusher which is positioned in the reservoir 308. The magnetic motor rotor 311 may be mechanically attached through the gear train 312 to affect translation of the plunger 310 within the reservoir 308. In operation, translation of the pusher 309 and plunger 310 within the reservoir (e.g., via actuation of the magnetic motor rotor 311) causes fluid (e.g., medicament) to be driven out of the reservoir 308 and through an infusion cannula to be delivered to a user at a subcutaneous treatment site.

The reservoir 308 may be prefilled with a medicament. The medicament, for example, can be U-100 insulin or U-500 insulin or other concentrations of insulin to suit different user use profiles, or may be user-fillable by way of a fill port (not shown). In those cases where the reservoir 308 is filled by the user, the user may completely fill the reservoir to capacity with medicament, or the user may choose to introduce less medicament and not completely fill the reservoir. Since an unknown amount of medicament may be injected into a user-filled reservoir, a plunger-pusher zeroing procedure (or "plunger seek") may be user-initiated or may be an automatic aspect of pump operation. A plunger seek procedure precisely determines and/or sets, before any medicament dispensing, exactly how far the pusher travels before it engages the plunger, enabling a calculation to determine the amount of medicament in the reservoir and, therefore, an estimate of time-to-empty and time for disposable assembly replacement. Additional details regarding the plunger seek procedure can be found in commonly owned U.S. Application No. 17,454,600, which is hereby incorporated by reference in its entirety.

The insertion assembly 400 is configured to insert both an infusion cannula 402 and a sensor electrode 404 out of the housing 302 via a first port 406 and a second port 408, respectively. Once inserted into the user's skin, the infusion cannula 402, which is fluidically coupled to the reservoir 308, can deliver medicament (e.g., insulin) therethrough to the user. Additionally, once the sensor electrode 404 is inserted into the user's skin, the sensor electrode can detect one or more physiological parameters, such as glucose levels in the intradermal and/or subcutaneous space. As will be known to one of ordinary skill in the art, measurements of glucose obtained in intradermal and/or subcutaneous space can be used to derive or infer blood glucose levels of the user. Such physiological parameters obtained via the sensor electrode 404 can optionally be used to control delivery of medicament via the infusion cannula 402. For example, as a user's glucose rises above a predetermined threshold (as determined on readings obtained via the sensor electrode 404), the magnetic motor rotor 311 can be initiated (e.g., by the electronics in the durable assembly 200) to drive the plunger 310 an appropriate amount to dispense medicament from the reservoir 308 into the user's body via the infusion cannula 402.

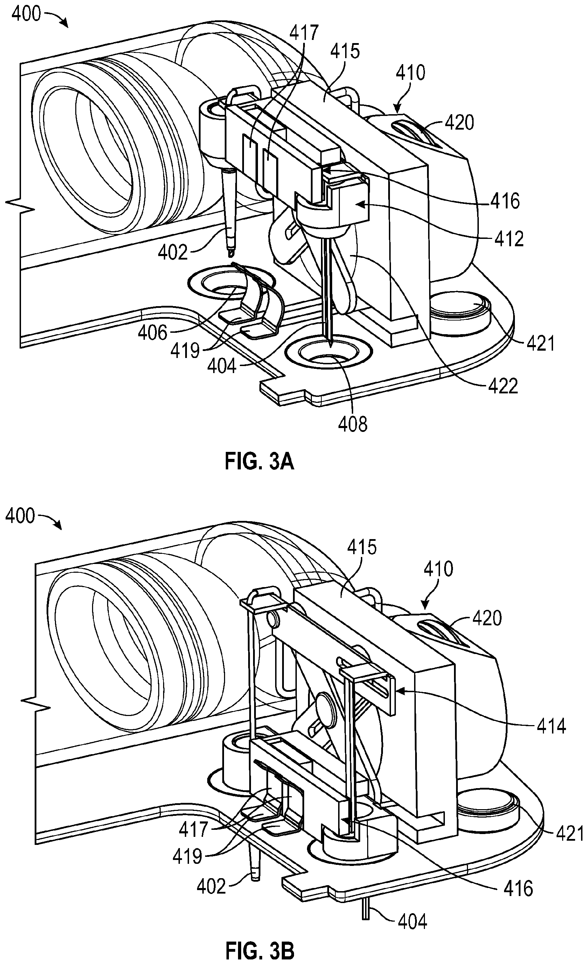

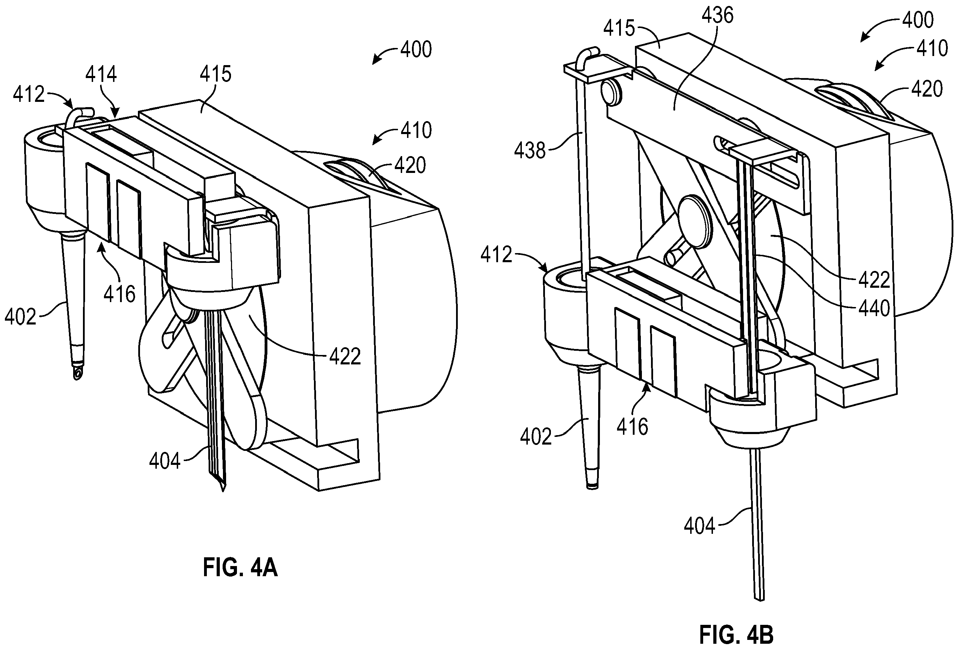

FIGS. 3A and 3B illustrate perspective views of the insertion assembly 400 in an unfired and fired position, respectively, and FIGS. 4A and 4B illustrate enlarged perspective views of the insertion assembly 400 in the unfired and fired positions, respectively. With reference to FIGS. 3A-4B together, the insertion assembly 400 includes a drive assembly 410, a carrier assembly 412, a trocar assembly 414, and a sensor electronics assembly 416. The carrier assembly 412 can be coupled to both the infusion cannula 402 and the sensor electrode 404, and the trocar assembly can be configured to releasably mate with the carrier assembly to move the carrier assembly from an unfired state (as shown in FIGS. 3A and 4A), downwardly to an inserted state (as shown in FIGS. 3B and 4B). As illustrated, in the unfired state (FIGS. 3A and 4A), both the infusion cannula 402 and the sensor electrode 404 are retracted so as to be disposed within the housing 302 (not shown in FIGS. 3A-4B). In the fired state (FIGS. 3B and 4B), both the infusion cannula 402 and the sensor electrode 404 extend through apertures 406 and 408, beyond the housing 302, and inserted into the user's skin.

As used herein with respect to a drive assembly, an "unfired state" can refer to a state in which a drive assembly has not yet moved either the trocar assembly or carrier assembly downwardly out of the housing and into a patient's skin. An "inserted state" can refer to an intermediate position in which both the trocar assembly and the carrier assembly have been moved downwardly, out of the housing, and into the patient's skin. A "fully fired state" can refer to a terminal position in which the drive assembly has retracted the trocar assembly upwardly and out of the patient's body while leaving the carrier assembly (including the sensor and cannula) extending beyond the housing and/or into the patient's body.

In various examples, the lateral distance between the infusion cannula 402 and the sensor electrode 404 can be selected to provide appropriate performance once inserted within the user's skin. If the lateral distance is too small, then the medicament provided via the infusion cannula 402 may interfere with physiological measurements obtained via the sensor electrode 404. On the other hand, having too great a lateral separation may lead to the device 100 being undesirably large and bulky. In various embodiments, the lateral separation between the infusion cannula 402 and the sensor electrode 404 can be at least about 10 mm (e.g., about 14 mm). Moreover, in some embodiments it may be desirable to insert the infusion cannula 402 and/or the sensor electrode 404 to a certain depth beneath the surface of the user's skin. If the infusion cannula 402 is not sufficiently deep, the medicament may not be effectively delivered to the user. Additionally or alternatively, if the sensor electrode 404 is not sufficiently deep, the physiological measurements may be unreliable. In various embodiments, the insertion depth (e.g., the distance from the further tip of the infusion cannula 402 and/or the sensor electrode 404 with respect to a lower surface of the housing when in the inserted state) may be at least about 6-10 mm or more. Achieving both sufficient lateral separation and sufficient insertion depth presents certain design challenges, particularly while trying to maintain a compact form factor for the device 100. Various embodiments of the drive assembly 410 disclosed herein may achieve both the desired lateral separation and the desired insertion depth for the infusion cannula 402 and the sensor electrode 404.

As described in more detail below, in operation the drive assembly 410 can be actuated to cause movement of both the trocar assembly 414 and the carrier assembly 412 downwardly from the unfired state (as shown in FIGS. 3A and 4A) to urge the carrier assembly 412 to the inserted state (as shown in FIGS. 3B and 4B). For example, the trocar assembly 414 can abut or otherwise be coupled to the carrier assembly 412 such that downward movement of the trocar assembly 414 results in downward movement of the carrier assembly 412, while upward movement of the trocar assembly 414 does not necessarily result in upward movement of the carrier assembly 412. In some embodiments, once the carrier assembly 412 is in the inserted state, the drive assembly 410 continues to move the trocar assembly 414 such that the trocar assembly 414 is retracted upward, while the carrier assembly 412 remains extended with the cannula 402 and the sensor electrode 404 extending beyond the housing 302 and into the user's skin. By this continued upward movement of the trocar assembly 414, the insertion assembly 400 moves to the fully fired position shown in Figures 3B and 4B.

The sensor electronics assembly 416 can be coupled to the carrier assembly 414 such that the two move downward together from the unfired position (FIGS. 3A and 4A) to the fired position (FIGS. 3B and 4B). The sensor electronics assembly 416 can include one or more electronic components configured to facilitate obtaining physiological measurements via the sensor electrode 404. This can include, for example, suitable analog or digital components, circuitry, processors, transceivers, etc. In the illustrated embodiment, the sensor electronics assembly 416 includes first electrical contacts 417 that can mate with corresponding second electrical contacts 419 when the carrier assembly 414 is moved from the unfired position (FIG. 3A) to the fired position (3B). This movement can place the sensor electronics assembly 416 into electrical communication with the sensor battery 421 via the mated first and second electrical contacts 417, 419. By only establishing electrical communication with the battery 421 upon firing the insertion assembly 400, the power can be preserved until the device 100 is in operation and the sensor electrode 404 has been inserted into the user's skin.

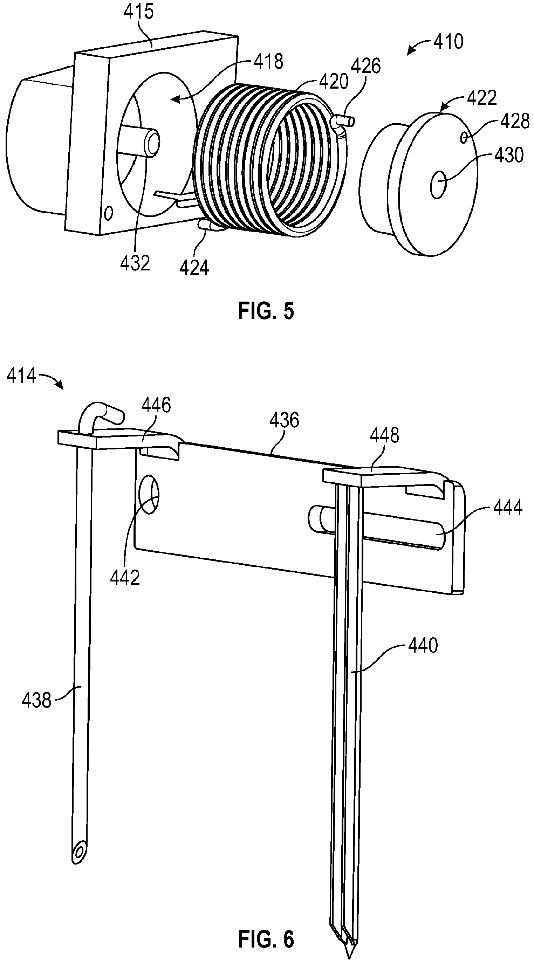

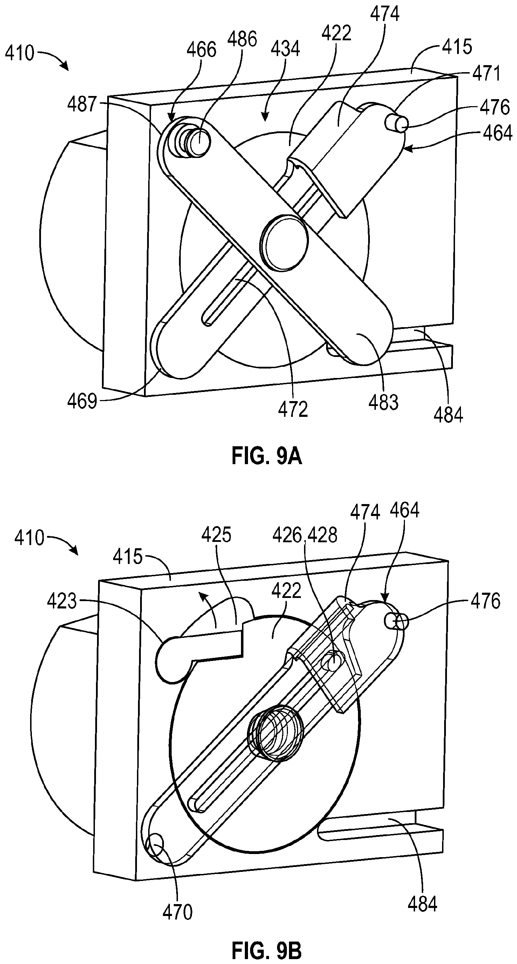

As noted above, it is the drive assembly 410 that causes the carrier assembly 412, trocar assembly 414, and sensor electronics assembly 416 to move from the unfired to the inserted position. As best seen in FIG. 5, the drive assembly 410 can include a drive assembly housing 415, a receptable 418 receiving a torsion spring 420 therein, and a drive wheel 422. The torsion spring 420 can take the form of a helical spring that include a first end crank pin 424 that is received within a corresponding receptable (not shown) in the drive assembly housing 415, and a second end crank pin 426 that extends within an opening 428 in the drive wheel 422, such that rotation of the second end crank pin 426 of the torsion spring 420 causes the drive wheel 422 to rotate in conjunction. The drive wheel 422 can also include a central aperture 430 configured to receive a central bearing shaft 432 therein. In the assembled configuration, the torsion spring 420 can be wound into a compressed state in which the torsion spring 420 is biased to unwind and cause rotation of the drive wheel 422 (or alternatively it can be wound into an extended state in which the torsion spring 420 is biased to wind to cause rotation of the drive wheel 422). Whether in the compressed or extended state, the torsion spring 420 can be releasably locked (e.g., in a cocked configuration) such that rotational movement is prohibited until a trigger mechanism (see FIG. 9B) is activated. For example, a manual trigger mechanism (e.g., depressing a button or lever), or a remote trigger mechanism (e.g., sending a wireless signal to an actuatable release mechanism) can cause the rotational movement to be permitted, such that the torsion spring 420 rotates, thereby causing the drive wheel 422 to rotate as well. As described in more detail below, the drive wheel 422 can be coupled to a scissor assembly 434, which in turn is coupled to the trocar assembly 414 and translates the rotational movement of the drive wheel 422 to axial (e.g., downward and upward) movement of the trocar assembly 414, which in turn causes axial (e.g., downward) movement of the carrier assembly 412.

As noted above, the trocar assembly 414 can be used to drive both the infusion cannula 402 and the sensor electrode 404 downwardly and beyond the housing 302 of the device 100. As best seen in FIG. 6, the trocar assembly 414 can include a trocar link 436 which extends laterally, and a first trocar 438 and a second trocar 440 each extending downwardly away from the trocar link 436. The trocar link 436 can be coupled to the scissor assembly 434 via an engagement hole 442 and cam slot 444 as described elsewhere herein, such that movement of the scissor assembly 434 (caused by rotation of the drive wheel 422), causes the trocar link 436 to move axially downward, thereby moving the first and second trocars 438, 440, axially downwardly. The trocar link 436 can include a first extension 446 that is coupled to the first trocar 438 and a second extension 448 that is coupled to the second trocar 440. These extensions 446, 448 can serve as mating surfaces that abut an upper surface of the carrier assembly 412, thereby causing the carrier assembly 412 to be moved downward in response to downward movement of the trocar assembly 414.

The first trocar 438 can be configured to releasably engage with the infusion cannula 402, such as by extending into a lumen of the infusion cannula 402. The second trocar 440 can be configured to releasably engage with the sensor electrode 404, such as by including a slot or recess in which the sensor electrode 404 is removably disposed. One or both of the trocars 438, 440 can have a sharp and/or pointed tip to facilitate puncturing the patient's skin. In various embodiments, the first and second trocars 438, 440 can be made of metal or other rigid material, such that the first and second trocars 438, 440 have sufficient structural integrity to be driven into the user's skin, carrying the infusion cannula 402 and sensor electrode 404 into the skin with them. Once the infusion cannula 402 and the sensor electrode 404 have been fully inserted into the user's skin, the trocar link 436 is moved upwardly (e.g., due to continued rotation of the drive wheel 422). Because the first and second trocars 438, 440 are releasably engaged with the infusion cannula 402 and the sensor electrode 404, respectively, the infusion cannula 402 and the sensor electrode 404 remain in place within the user's skin (e.g., in the inserted position as shown in FIGS. 3B and 4B) while the first and second trocars 438, 440 are retracted upwardly. The first trocar 438 can be fluidically coupled to the cannula 402 such that, while the first trocar 438 is retracted, it is not completely released from the cannula and then delivers fluid into the cannula 402. Although trocars are referred to herein, any suitable elongated member can be used to releasably mate with the infusion cannula 402 and/or the sensor electrode 404. Such elongated members can be, for example, a stylet, rod, shaft, tube, needle, or any other suitable configuration.

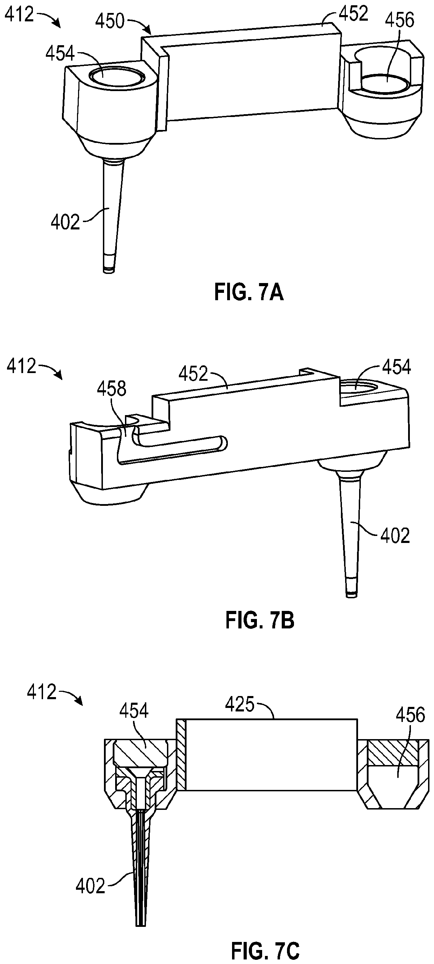

FIGS. 7A-7C illustrate front perspective, rear perspective, and cross-sectional views, respectively, of the carrier assembly 412. As noted previously, the carrier assembly 412 can be releasably mated with trocar assembly 414 such that downward movement of the trocar assembly 414 causes downward movement of the carrier assembly 412. The carrier assembly 412 can include a cannula carrier 450, which extends laterally and includes an upper mating surface 452 configured to engage the extensions 446, 448 of the trocar assembly 414 (FIG. 6). The carrier assembly 412 further includes a cannula septum 454 that overlies and seals the cannula 402 extending downwardly therefrom. The cannula septum 454 can maintain a hermetic and/or substantially air-tight seal over the cannula 402 while being pierced by the first trocar 438. The first trocar 438 can be in fluid communication with the reservoir such that, when disposed within the lumen 455 of the cannula 402, the cannula 402 is in fluid communication with the reservoir. A sensor septum 456 is disposed on an opposite end of the carrier 450, and is configured to overlie the sensor electrode extending downwardly therefrom (not shown in FIGS. 7A-7C for clarity). The septum 456 provides a fluid seal around the sensor electrode to seal out the external environment (e.g., water) from getting into the pump around the sensor electrode and/or the second trocar. As seen in FIG. 7B, the carrier assembly 412 includes a cam slot 458 configured to engage with a pin of the first link of the scissor assembly (described below with respect to 9A-10C).

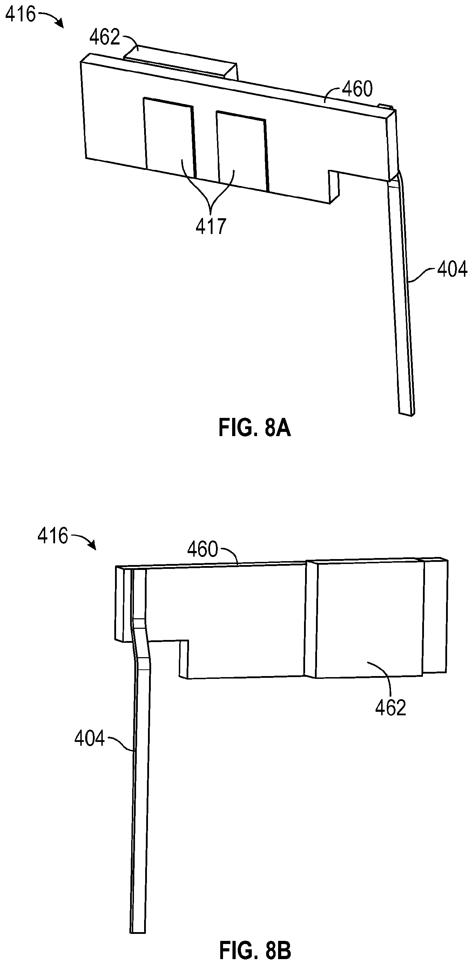

As described previously, the carrier assembly 412 can be coupled to the sensor electronics assembly 416. FIGS. 8A and 8B illustrate front and rear perspective views, respectively, of the sensor electronics assembly 416. In the illustrated embodiment, the sensor electronics assembly 416 includes a printed circuit board or other substrate 460 on which the first electrical contacts 417 are disposed. As noted previously, these first electrical contacts 417 can mate with corresponding electrical contacts 419 of the device 100 when the carrier assembly 414 is moved from the unfired position to the inserted position (FIGS. 3A and 3B). The sensor electronics assembly 416 can further include one or more components 462 coupled to the substrate 460. Such component(s) 462 can include, for example, low power wireless communication components, data processing circuitry, processing circuitry, data storage, or any other suitable components.

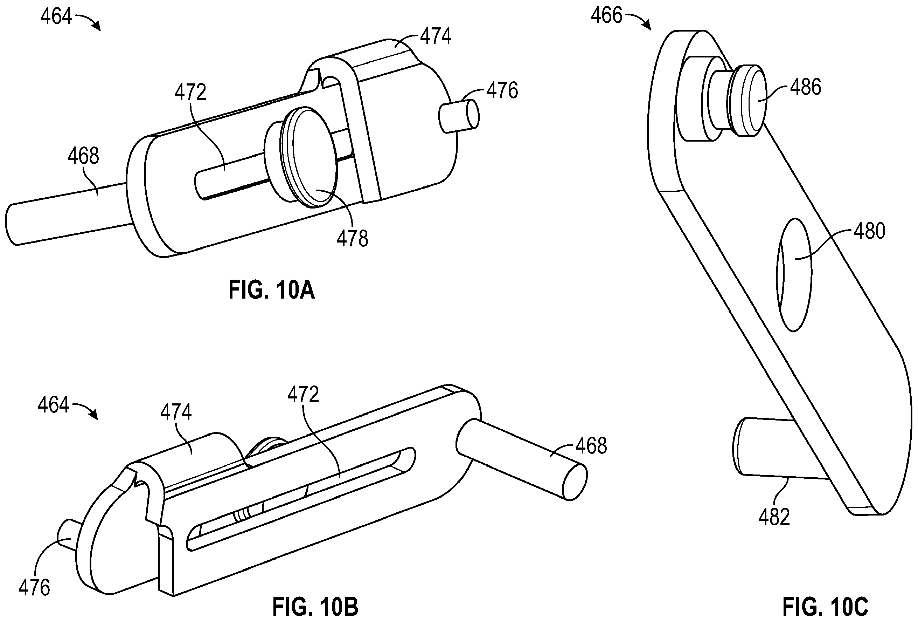

FIG. 9A illustrates a perspective view of the drive assembly 410 including the scissor assembly 434. The scissor assembly includes a first link 464 and a second link 466. FIG. 9B illustrates a perspective view of the drive assembly 410 including the scissor assembly 434, with the second link 466 omitted and the first link 464 shown in transparency. FIG. 10A and 10B illustrate front and rear perspective views, respectively, of the first link 464, and FIG. 10C illustrates a perspective front view of the second link 466. Referring to FIGS. 9A-10C together, the first link 464 includes a first pin 468 at its first end 469 that is configured to be received within the bearing hole 470 within the drive assembly housing 415. The first link 464 can thereby pivot about this point between a first, expanded configuration (as illustrated in FIGS. 9A and 9B), and a second, collapsed configuration in which the first link 464 and the second link 466 are oriented more parallel to one another than in the expanded configuration. The first link 464 further includes a cam slot 472 extending along a length of the first link 464. The cam slot 472 is configured to receive the crank pin 426 that extends through the opening 428 in the drive wheel 422. In this configuration, rotation of the torsion spring (not shown in these views) causes the crank pin 426 to rotate, thereby pulling the second end 471 of the first link 464 downwardly into the collapsed configuration. The first link further includes a wrap portion 474 configured to receive the second link 466 therein when the scissor assembly 434 is in the collapsed configuration. The wrap portion 474 can include a portion configured to extend over an upper surface of the second link 466 when in the collapsed configuration, and can further include a pin 476 configured to slidably engage the cam slot 444 of the trocar link (FIG. 6). In this configuration, rotational movement of the first link 464 causes downward axial movement of the trocar assembly 414 (FIG. 6).

The first link 464 can be joined to the second link 466 via a bearing 478 that mates with a corresponding aperture 480 in the second link 464. The bearing 478 and aperture 480 can each be disposed along a central portion of the respective first link 464 and second link 466. The second link 464 can further include a pin 482 at a first end 483 configured to engage a bearing slot 484 of the drive assembly housing 415. As best seen in FIG. 9A, the bearing slot 484 permits slidable movement of the pin 482, thereby enabling the second link 466 to move from the expanded configuration (shown in FIG. 9A) to a collapsed configuration in which the pin 482 extends further along the bearing slot 484 toward an outer surface of the drive assembly housing 415. The second link 466 can further include a second pin 486 on a second end 487 opposite the first and extending in a forward direction. The second pin 486 can be configured to mate with the engagement hole 442 of the trocar assembly 414 (FIG. 6). In operation, downward movement of the first link 464 causes downward movement of the second link 466 via the bearing 478. This downward movement of the second link 466 causes the pin 486 to urge the trocar assembly 414 (FIG. 6) downwardly.

As shown in FIG. 9B, the drive wheel 422 can include a recess 425 configured to releasably engage with a latch mechanism 423. In operation, the torsion spring can be placed in a tightly wound, compressed state, and the latch mechanism 423 can be positioned within the recess 425 to prohibit the drive wheel 422 from rotating. To release the drive wheel, the latch mechanism 423 can be rotated upwardly or otherwise disengaged from the recess 425, thereby permitting the drive wheel 422 to rotate as the torsion spring unwinds. In various examples, the latch mechanism 423 can be manually triggered (e.g., via a mechanical button or other actuator), or may be remotely triggered (e.g., via an electrical signal provided by a controller to release the latch mechanism 423).

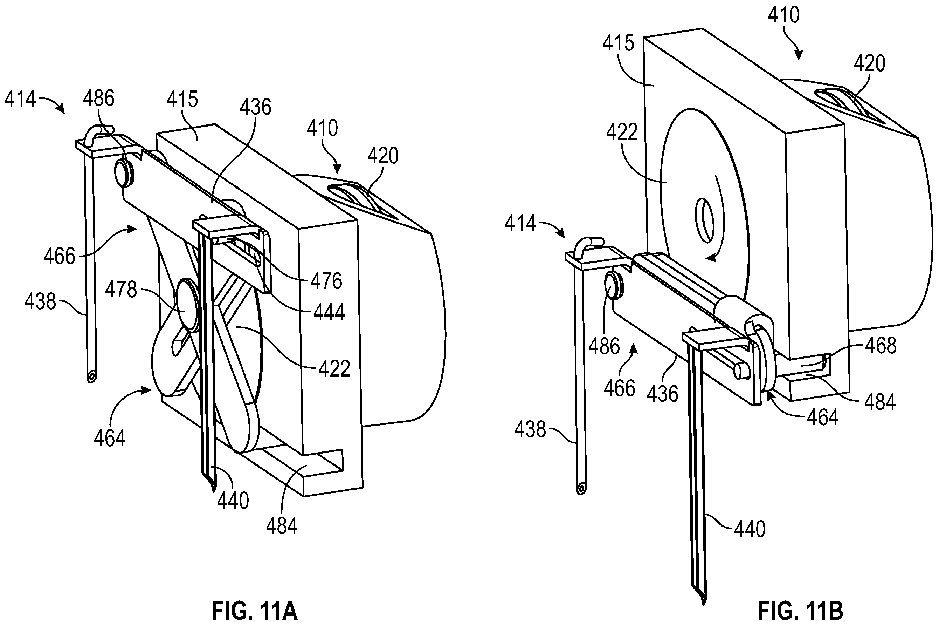

FIGS. 11A and 11B illustrate front perspective views of the drive assembly 410 coupled to the trocar assembly 414 in an unfired and inserted position, respectively. The inserted position can represent an intermediate stage of the drive assembly 410, in which the trocar carrier assembly 414 has been moved downwardly to insert the trocars into the body (thereby inserting the sensor electrode and cannula into the body). Following this intermediate position, the drive assembly 410 can further move the trocar carrier assembly 414, now in the upward direction to retract the trocars from the patient's body. As illustrated, the pin 476 of the first link 464 is slidably received within the cam slot 444 of the trocar link 436, and the pin 486 of the second link 466 is rotatably mated with the engagement hole 442 of the trocar link 436. As the torsion spring 420 rotates, the crank pin 426 causes the first link 464 to rotate such that the pin 476 exerts a downward force on the trocar link 436 while also sliding laterally within the cam slot 444. Additionally, this rotational movement of the first link 464 exerts a downward force on the second link via the bearing 478. As the second link 466 rotates, the pin 468 of the second link 466 slides within the bearing slot 484 until the first link 464 and the second link 466 are in the collapsed configuration illustrated in FIG. 11B. In the collapsed configuration, the first link 464 and the second link 466 may extend along axes substantially parallel to one another. In some embodiments, in the collapsed (inserted) configuration the first link 464 and the second link 466 may not be parallel, but may nonetheless be nearer to parallel than in the expanded (unfired) configuration. As the torsion spring continues to rotate, the crank pin 426 continues to slide within the cam slot 427, causing the scissor assembly to return to an expanded, uncollapsed configuration, thereby retracting the trocar carrier assembly 414 upward while leaving the cannula carrier assembly 412 in the downwardly inserted configuration.

### III. Select Exemplary Devices with Dual-Crank Drive Assemblies

As described above, in an integrated infusion pump and sensor device, the insertion assembly can insert both an infusion cannula and a sensor electrode into a user's skin. In various embodiments, it may be desirable to maintain both sufficient lateral separation between the infusion cannula and the sensor electrode, and to provide sufficient insertion depth, without unduly increasing the overall size of the device. In addition to the scissor assembly described above with respect to FIGS. 1A-11B, in various embodiments a dual-crank drive assembly can be used to insert both an infusion cannula and a sensor electrode from a device housing into a user's skin.

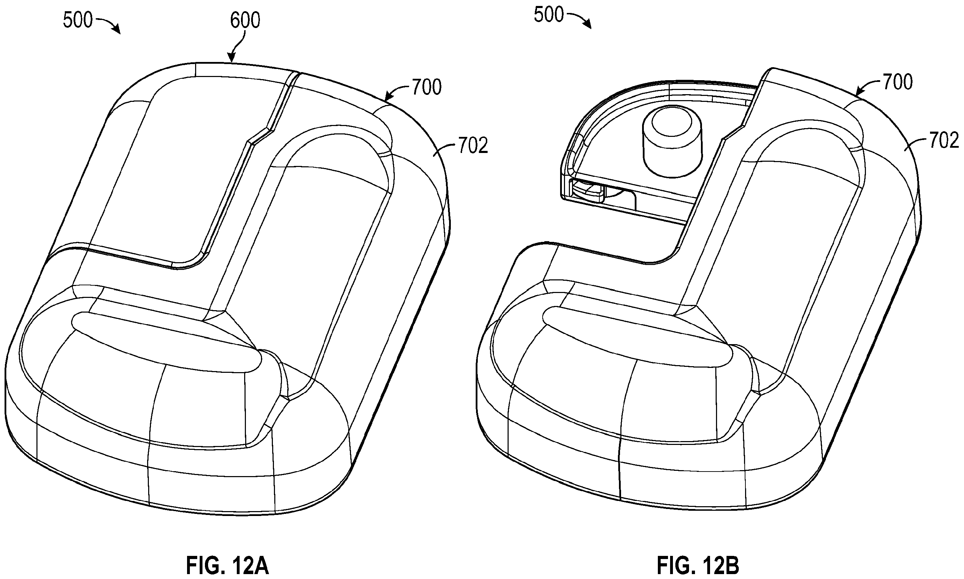

FIGS. 12A Is a perspective view of an infusion device with integrated sensor ("device 500") that includes a durable assembly 600 and a disposable assembly 700 in accordance with several embodiments of the present technology. FIG. 12B illustrates the device 500 with the durable assembly 600 removed. The device 500, durable assembly 600, and disposable assembly 700 can be generally similar to the device 100, durable assembly 200, and disposable assembly 300 described above. However, as described in more detail below, the disposable assembly 700 can include a drive assembly that includes multiple drive wheels, in contrast to the scissor assembly described above.

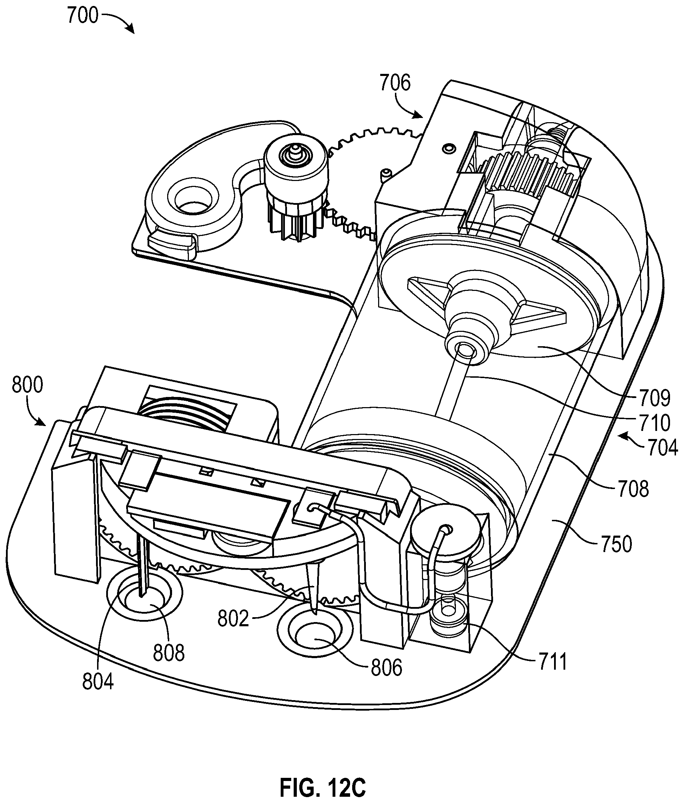

The disposable assembly 700, shown in more detail in FIG. 12C, may include a reservoir assembly 704 and an insertion assembly 800, each mounted on a baseplate 750. The reservoir assembly 704 can comprise a drive assembly 706, a reservoir 708, and a pusher 709 coupled to a plunger 710. The pusher 709 is coupled to the drive assembly, and both the pusher 709 and the plunger 710 can be contained within the reservoir 708. In operation, translation of the plunger 710 within the reservoir (e.g., via actuation of a magnetic motor) causes fluid (e.g., medicament) to be driven out of the reservoir 708 and through an infusion cannula to be delivered to a user at a subcutaneous treatment site. The reservoir 708 may be prefilled with a medicament. The medicament, for example, can be U-100 insulin or U-500 insulin or other concentrations of insulin to suit different user use profiles, or may be user-fillable by way of a fill port.

The insertion assembly 800 is configured to insert both an infusion cannula 802 and a sensor electrode 804 out of the housing 702 via a first port 806 and a second port 808, respectively. Once inserted into the user's skin, the infusion cannula 802, which is fluidically coupled to the reservoir 708 (e.g., through the trocar), can deliver medicament (e.g., insulin) therethrough to the user. Additionally, once the sensor electrode 804 is inserted into the user's skin, the sensor electrode 804 can detect one or more physiological parameters, such as glucose levels in the intradermal and/or subcutaneous space. As will be known to one of ordinary skill in the art, measurements of glucose obtained in intradermal and/or subcutaneous space can be used to derive or infer blood glucose levels of the user. These physiological parameters obtained via the sensor electrode 804 can optionally be used to control delivery of medicament via the infusion cannula 802. For example, as a user's glucose rise above a predetermined threshold (as determined on readings obtained via the sensor electrode 804), a magnetic motor can be initiated to drive the plunger 710 an appropriate amount to dispense medicament from the reservoir 708 into the user's body via the infusion cannula 802.

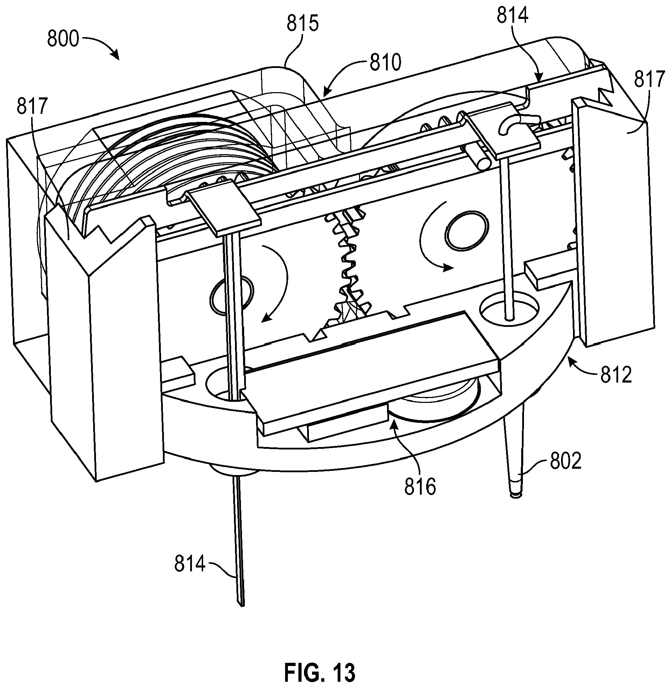

FIG. 13 is an enlarged perspective view of the insertion assembly 800 in the inserted position. As illustrated, the insertion assembly 800 includes a drive assembly 810, a carrier assembly 812, a trocar assembly 814, and a sensor electronics assembly 816. A slide guide 817 is positioned on opposing lateral sides of the trocar assembly 814 and the carrier assembly 812 may move upward and downward. The carrier assembly 812 can be coupled to both the infusion cannula 802 and the sensor electrode 804, and the trocar assembly 814 can be configured to releasably mate with the carrier assembly 812 to move the carrier assembly 812 from an unfired state downwardly to a inserted state (as shown in FIG. 13). In the unfired state (not shown), both the infusion cannula 802 and the sensor electrode 804 are retracted so as to be disposed within the housing 702 (not shown in FIG. 13 for clarity). In some examples, in the unfired state, the infusion cannula and sensor electrode can be arranged similarly to the configuration shown in FIG. 3B. In the inserted state (shown in FIG. 13), both the infusion cannula 802 and the sensor electrode 804 extend downwardly, and in operation extend into the user's skin.

In various examples, the lateral distance between the infusion cannula 802 and the sensor electrode 804 can be selected to provide appropriate performance once inserted within the user's skin. In various embodiments, the lateral separation between the infusion cannula 802 and the sensor electrode 804 can be at least about 10 mm or more (e.g., about 14 mm). Moreover, in some embodiments it may be desirable to insert the infusion cannula 802 and/or the sensor electrode 804 to a certain depth beneath the surface of the user's skin. In various embodiments, the insertion depth (e.g., the distance from the further tip of the infusion cannula 802 and/or the sensor electrode 804 with respect to a lower surface of the housing when in the inserted state) may be at least about 6-10 mm or more.

As described in more detail below, in operation the drive assembly 810 is actuated to cause movement of both the trocar assembly 814 and the carrier assembly 812 downwardly from the unfired state to urge the carrier assembly 812 to the inserted state (as shown in FIG. 13). For example, the trocar assembly 814 can abut or otherwise be coupled to the carrier assembly 812 such that downward movement of the trocar assembly 814 results in downward movement of the carrier assembly 812, while upward movement of the trocar assembly 814 does not result in upward movement of the carrier assembly 812. In some embodiments, once the carrier assembly 812 is in the inserted state, the drive assembly 810 continues to move the trocar assembly 814 such that the trocar assembly 814 is retracted upward, while the carrier assembly 812 remains extended with the infusion cannula 802 and sensor electrode 804 extending beyond the housing 702 and into the user's skin.

The sensor electronics assembly 816 can be coupled to the carrier assembly 814 such that the two move downward together from the unfired position to the inserted position. The sensor electronics assembly 816 can include one or more electronic components configured to facilitate obtaining physiological measurements via the sensor electrode 804. This can include, for example, suitable analog or digital components, a battery, circuitry, processors, transceivers, etc.

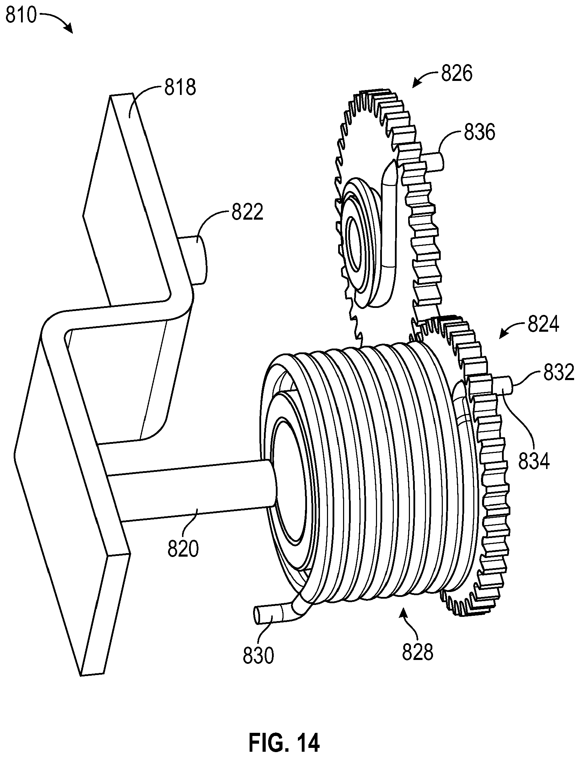

As noted above, it is the drive assembly 810 that causes the carrier assembly 812, trocar assembly 814, and sensor electronics assembly 816 to move from the unfired to the inserted position. The drive assembly 810 can include a drive assembly housing 815. As best seen in FIG. 14, which illustrates a partially exploded view of the drive assembly 810 with the housing 815 omitted, the drive assembly 810 includes a support bracket 818 with a first bearing shaft 820 and a second bearing shaft 822 extending therefrom. The first bearing shaft 820 is configured to receive a first drive wheel 824 thereon and the second bearing shaft 822 is configured to receive a second drive wheel 826 thereon. The first drive wheel 824 and the second drive wheel 826 can be mated together, for example having interlocking teeth such that rotation of the first drive wheel 824 causes corresponding rotation in the opposite direction of the second drive wheel 826. In various embodiments the coupling between the first drive wheel and the second drive wheel can take other forms.

In some embodiments, the first drive wheel 824 can take the form of a main drive gear and can be coupled to a torsion spring 828. The torsion spring 828 can comprise a helical coil having an anchor pin 830 and a first crank pin 832 that extends through an aperture 834 in the first drive wheel 824. In this configuration, rotation of the first crank pin 832 with respect to the support bracket 818 will cause rotation of the first drive wheel 824, which in turn will cause a corresponding opposite rotation of the second drive wheel 826. The second drive wheel 826 can take the form of an idle gear that is not directly coupled to a torsion spring. However, a second crank pin 836 may extend from the second drive wheel 826. As described in more detail below, rotation of the first and second drive wheels 824, 826 causes the first and second crank pins 832, 836 to move rotationally. When the first and second crank pins 832, 836 are each received within a cam slot of the trocar assembly 814, this rotational movement of the drive wheels can cause the trocar assembly 814 to move axially (e.g., upwardly and downwardly).

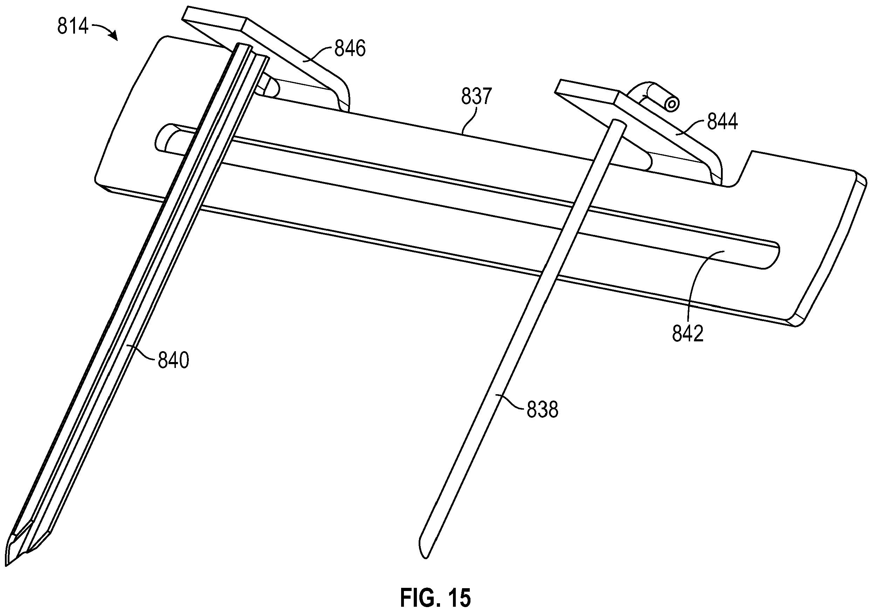

As noted above, the trocar assembly 814 can be used to drive both the infusion cannula 802 and the sensor electrode 804 downwardly and beyond the housing of the device. As best seen in FIG. 15, the trocar assembly 814 can include a trocar slider 837 which extends laterally, and a first trocar 838 and a second trocar 840 each extending downwardly away from the trocar slider 837. The trocar slider 837 can be coupled to the drive assembly 810 via a cam slot 842, such that movement of the crank pins 832, 836 (caused by rotation of the first and second drive wheels 824, 826), causes the trocar slider 837 to move axially downward, thereby moving the first and second trocars, 838, 840, axially downwardly. The trocar slider 837 can include a first extension 844 which is coupled to the first trocar 838 and a second extension 846 that is coupled to the second trocar 840. These extensions 844, 846 can serve as mating surfaces that abut an upper surface of the carrier assembly 812, thereby causing the carrier assembly 812 to be moved downward in response to downward movement of the trocar assembly 814.

The first trocar 838 can be configured to releasably engage with the infusion cannula 802, such as by extending into a lumen of the infusion cannula 802. The second trocar 840 can be configured to releasably engage with the sensor electrode 804, such as by including a slot or recess in which the sensor electrode 804 is removably disposed. One or both of the trocars 838, 840 can have a sharp and/or pointed tip to facilitate puncturing the patient's skin. In various embodiments, the first and second trocars 838, 840 can be made of metal or other rigid material, such that the first and second trocars 838, 840 have sufficient structural integrity to be driven into the user's skin, carrying the infusion cannula 802 and sensor electrode 804 into the skin with them. Once the infusion cannula 802 and the sensor electrode 804 have been fully inserted into the user's skin, the trocar slider 837 is moved upwardly (e.g., due to continued rotation of the drive wheels 824, 826). Because the first and second trocars 838, 840 are moveably engaged with the infusion cannula 802 and the sensor electrode 804, respectively, the infusion cannula 802 and the sensor electrode 804 remain in place within the user's skin (e.g., in the inserted position as shown in FIG. 13) while the first and second trocars 838, 840 are retracted upwardly. Although trocars are referred to herein, any suitable elongated member can be used to releasably mate with the infusion cannula 802 and/or the sensor electrode 804. Such elongated members can be, for example, a stylet, rod, shaft, tube, needle, or any other suitable configuration.

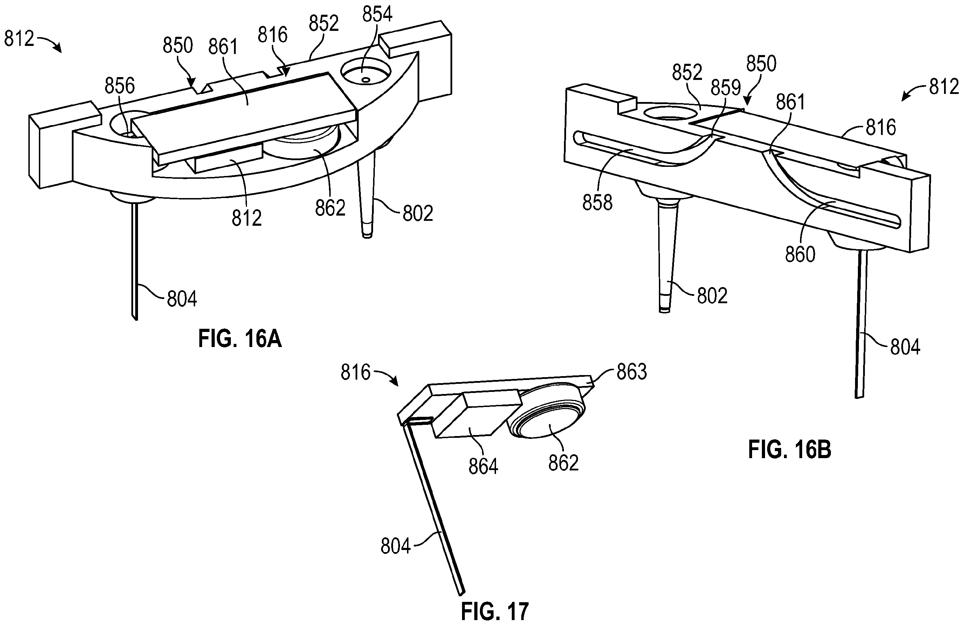

FIGS. 16A and 16B illustrate front and rear perspective views, respectively, of the cannula carrier assembly 812 with the electronics assembly 816 coupled thereto. FIG. 17 illustrates a perspective view of the electronics assembly 816 isolated. Referring to FIGS. 16A-17 together, as noted previously, the carrier assembly 812 can be releasably mated with trocar assembly 814 such that downward movement of the trocar assembly 814 causes downward movement of the carrier assembly 812 The carrier assembly 812 can include a cannula carrier 850, which extends laterally and includes an upper mating surface 852 configured to engage the extensions 844, 846 of the trocar assembly 814 (FIG. 15). The carrier assembly 812 further includes a cannula septum 854 that overlies and seals the cannula 802 extending downwardly therefrom. The cannula septum 854 can maintain a hermetic and/or substantially air-tight seal over the cannula 802 and around the first trocar 838, which pierces the septum 854. Additionally, the cannula 802 can have a lumen that is placed in fluid communication with the reservoir via the first trocar 838, which is fluidically coupled to the reservoir, even after upward movement of the trocar assembly. A sensor septum 856 is disposed on an opposite end of the carrier 850, and is configured to overlie the sensor electrode 804 extending downwardly therefrom. As seen in FIG. 16B, the carrier assembly 812 includes first and second cam slots 858, 860 configured to engage with the first and second crank pins 832, 836 of the drive assembly 810 during the downstroke. Upon continued rotation of the drive wheels 824, 826 and therefore the crank pins 832, 836, the crank pins move out of the first and second cam slots 858, 860 at the open ends 859, 861 of the cam slots 858, 860 without lifting the carrier assembly 812 away from the inserted position.

As illustrated, the carrier assembly 812 can be coupled to the sensor electronics assembly 816. In the illustrated embodiment, the sensor electronics assembly 816 includes a printed circuit board or other substrate 863 to which a battery 862 and one or more components 864 are coupled. Such component(s) 864 can include, for example, low power wireless communication components, data processing circuitry, processing circuitry, data storage, or any other suitable components.

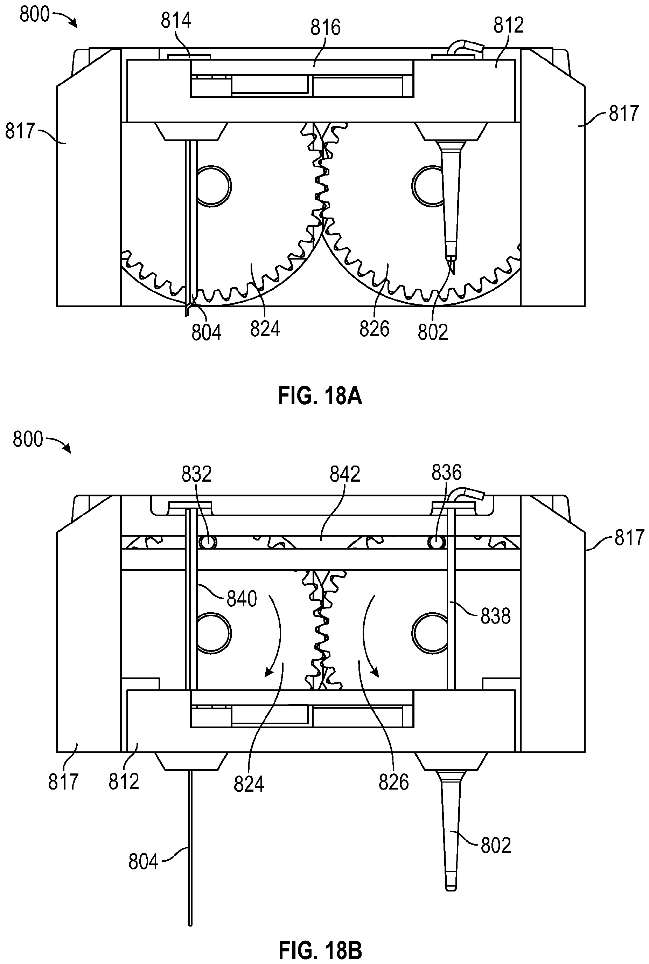

FIGS. 18A and 18B illustrate end views of the insertion assembly 800 in an unfired and inserted position, respectively. As illustrated, in the unfired position (FIG. 18A), the cannula carrier assembly 812, which carries the infusion cannula 802 and the sensor electrode 804, is in a retracted position such that the infusion cannula 802 and the sensor electrode 804 are disposed within the housing of the device. By rotating the first and second drive wheels 824, 826 (e.g., via rotation of the torsion spring (not shown) which is coupled to the first drive wheel 824), the first and second crank pins 832, 836 engage the cam slot 842 of the trocar assembly 814 and urge the trocar assembly 814 downwardly. This downward motion of the trocar assembly 814 causes the carrier assembly 812 (and the electronics assembly 816, which is coupled thereto) to move downward to the inserted position shown in FIG. 18B. After firing downward, the trocar assembly 814 can be retracted upwardly (e.g., by continued rotation of the drive wheels 824, 826), leaving the infusion cannula 802 and the sensor electrode 804 projecting out and into the user's skin. Because the trocar is fluidically coupled to the reservoir and the cannula, fluid can thereafter be delivered as needed through the infusion cannula 802 and into the user's body. In various examples, the device can be fired as described above by use of a release mechanism that is either mechanically actuated (e.g., using a button, switch, etc.) or remotely actuated (e.g., via wireless transmission).

### IV. Select Exemplary Embodiments of Multi-Sensor Devices

As described above, in an integrated infusion pump and sensor device, a disposable component can include a blood glucose sensor electrode. In various exemplary embodiments, such a device can additionally or alternatively include one or more other sensors. Data collected via such sensor(s) can be transmitted wirelessly from the disposable assembly to the durable assembly as noted previously. Among examples, the additional sensors can include hydraulic sensors to detect fluid flow throughout the assembly, plunger position sensors to detect a position of the plunger within the reservoir, mechanical sensors to detect position, orientation, strain, or other parameters of the mechanical components such as the drive assembly that moves the plunger, temperature sensors, pressure sensors, or any other suitable sensors. In various examples, the sensors can be used to evaluate pump performance (e.g., discharge pressure, bubbles, flowrate, plunger position, plunger force, leadscrew angular position) or any other desired parameters of the device.

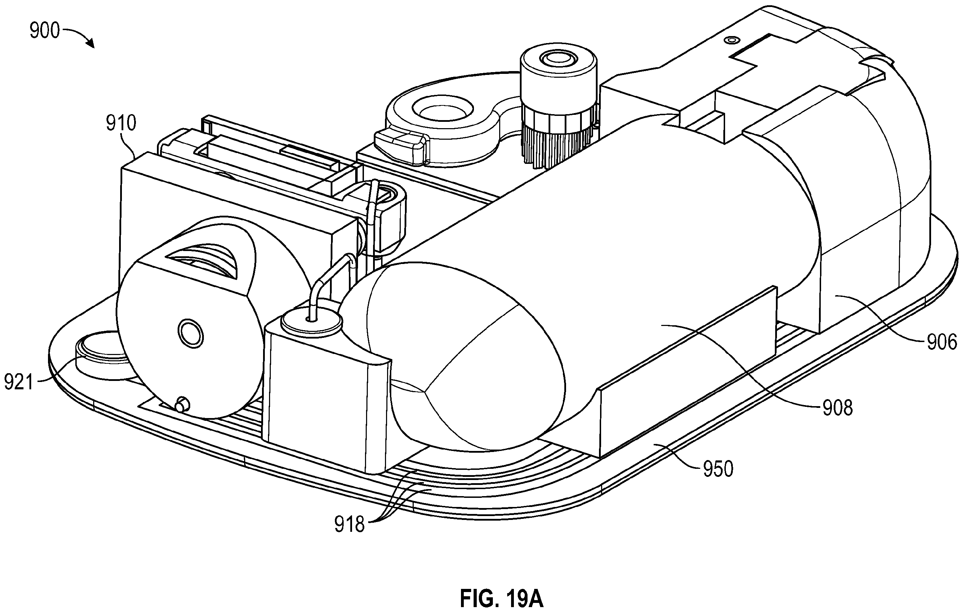

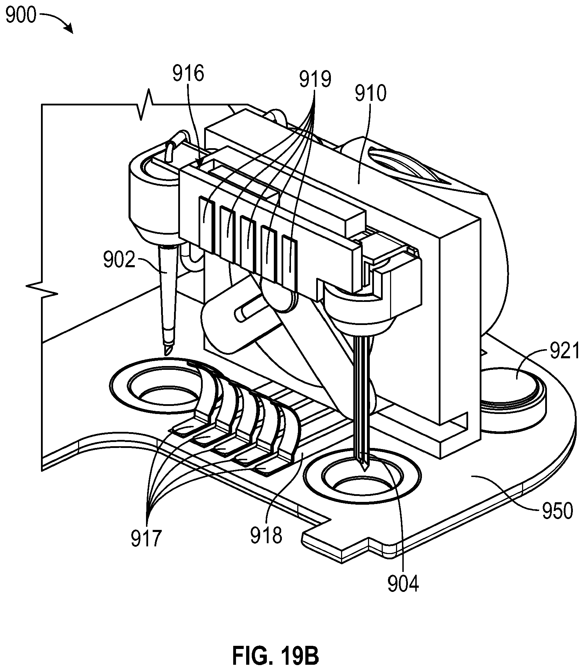

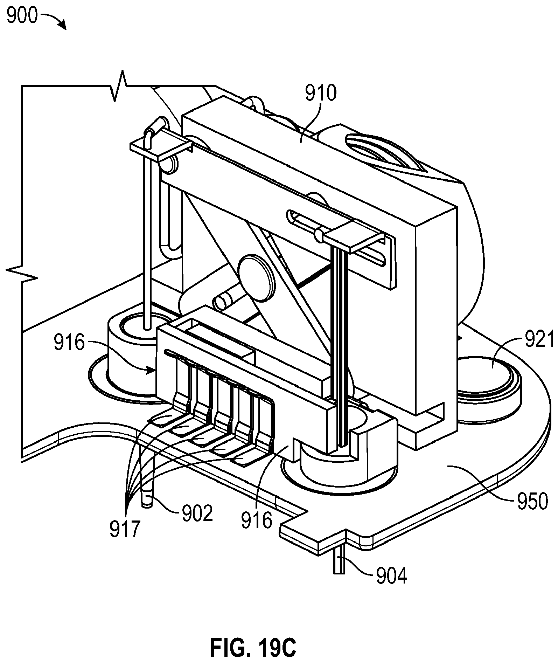

Figures 19A-19C illustrate several views of a disposable assembly 900 of such a multi-sensor device with a top shell removed for clarity. In some embodiments, the disposable assembly 900 can include some or all of the features of the disposable assembly 300 and the disposable assembly 700 described elsewhere herein, except that the disposable assembly 900 can include one or more additional sensors or sensor components. Figure 19A shows a side perspective view of the disposable assembly 900, Figure 19B shows an enlarged detail view of a portion of the assembly 900 in an unfired position, and Figure 19C shows an enlarged detail view of the portion of the assembly 900 in a downwardly inserted position. In the illustrated embodiment, the disposable assembly 900 includes a scissor drive assembly 910 to effect movement of the sensor electrode 904 and cannula 902. However, the various sensors described herein can be equally used in a disposable assembly having other drive mechanisms, such as the dual-crank drive assembly described elsewhere herein.

As shown in Figure 19A, a plurality of electrical traces 918 can extend along the baseplate 950 on which the various components of the disposable assembly 900 are mounted. At least one of these traces 918 can be electrically connected to the battery 921, and one or more of the traces 918 can be in electrical communication with one or more sensors disposed about the disposable assembly 900. Such sensors can include, for example, mechanical sensors (e.g., disposed within the plunger drive assembly 906), plunger position sensors (e.g., disposed within or about the reservoir 908), hydraulic sensors (e.g., disposed within or about any of the fluid paths between the reservoir 908 and the cannula 902) or any other suitable sensors or sensor components. Some or all of the electrical traces 918 can also be in electrical communication with corresponding electrical contacts 917.

As best seen in Figures 19B and 19C, when the sensor electronics assembly 916 is moved from the unfired position (shown in Figure 19B) to the downwardly inserted position (shown in Figure 19C), the electrical contacts 919 of the sensor electronics assembly 916 are placed into direct contact with the electrical contacts 917, which are in turn coupled to the traces 918. In this connected state (shown in Figure 19C), power can be provided to the various sensors as well as to the other electrical components of the sensor assembly 916 (e.g., components for signal processing, wireless transmission, etc.). In operation, data from these various sensors can be processed as required by the on-board electronics then wirelessly transmitted to the durable assembly (not shown) for further processing. Although the illustrated example depicts five traces, in various embodiments the number of traces and/or electrical contacts can be more or less, depending on the desired configuration, number and type of sensors, etc.

### V. Conclusion

Although the devices and methods are described in the context of automatic cannula insertion and patch pumps, it should be appreciated that the techniques are equally applicable to a variety of medical devices (e.g., infusion ports) and to a variety of at least partially implantable devices (e.g., sensors). It should also be noted here that the specification describes structures and methods that are especially well-suited for the subcutaneous delivery of high concentration insulin (i.e., U-200 insulin and above) such as U-500 insulin as well as lower concentration insulin such as U-100 insulin. Nevertheless, it should be appreciated that the present inventions are applicable to a wide variety of infusion pumps and medicaments. For example, the present inventions are also applicable to medicaments such as, for example, drugs to mask pain, chemotherapy and other cancer related drugs, antibiotics, hormones, GLP-1, glucagon, various other drugs that include large molecules and proteins that may require a high level of delivery accuracy, as well as to relatively high concentration insulin (i.e., U-200 insulin and above) such as U-500 insulin, as well as lower concentration insulin, such as U-100 insulin.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Integrated sensor and infusion devices are disclosed herein. The present technology includes, for example, an integrated sensor and infusion device for sensing physiological parameter(s) and delivering a medicament to a body of a user based at least in part on the sensed parameter(s). The device can comprise an insertion assembly comprising a carrier assembly comprising a cannula carrier, a trocar assembly removably coupled to the carrier assembly, and a drive assembly comprising a torsion spring coupled to the trocar assembly such that, when actuated, the torsion spring rotates to drive the trocar assembly and the carrier assembly axially downward to insert an infusion cannula and sensor electrode into a user's skin. The drive assembly can comprise a plurality of coupled drive wheels and/or a scissor assembly with multiple interacting links.

Further disclosed herein is the subject-matter of the following clauses:
1. An integrated sensor and infusion device, the device comprising:
   a reservoir assembly comprising a reservoir configured to retain medicament therein;
   a sensor electronics assembly configured to receive signals from a sensor; and
   an insertion assembly comprising:
      a carrier assembly comprising a cannula carrier, an infusion cannula extending downwardly away from the cannula carrier, and a sensor electrode extending downwardly away from the cannula carrier at a position laterally spaced apart from the infusion cannula, wherein the infusion cannula is fluidically coupled to the reservoir;
      a trocar assembly comprising a trocar link, a first trocar configured to removably engage the infusion cannula, and a second trocar configured to removably engage the sensor electrode, the trocar assembly removably coupled to the carrier assembly; and
      a drive assembly comprising a torsion spring coupled to the trocar assembly such that, when actuated, the torsion spring rotates to drive the trocar assembly and the carrier assembly axially downward to insert the first trocar, the infusion cannula, the second trocar, and the sensor electrode into a user's skin.
2. The device of clause 1, wherein each of the components of the device are configured to be housed within a device housing prior to insertion of the first trocar, the infusion cannula, the second trocar, and the sensor electrode into the user's skin, and wherein the first trocar, the infusion cannula, the second trocar, and the sensor electrode extend out of the housing for insertion into the user's skin.
3. The device of any one of clauses 1-2, wherein a first amount of rotation by the torsion spring drives the first trocar, the infusion cannula, the second trocar, and the sensor electrode into the user's skin, and wherein a second amount of rotation retracts the first trocar and the second trocar from the user's skin while leaving the infusion cannula and the sensor electrode in the user's skin.
4. The device of clause 3 or of any of the preceding clauses, wherein the first amount of rotation is approximately 180 degrees, and the second amount of rotation is an additional approximately 180 degrees.
5. The device of any one of clauses 1-4, wherein the torsion spring is actuated via a manual trigger mechanism.
6. The device of any one of clauses 1-4 or of any of the preceding clauses, wherein the torsion spring is actuated via a remote trigger mechanism.
7. The device of any one of clauses 1-6, wherein the first trocar is configured to extend within a lumen of the infusion cannula.
8. The device of any one of clauses 1-7, wherein the sensor electrode is configured to be removably received within a recess of the second trocar.
9. The device of any one of clauses 1-8, wherein the drive assembly further comprises a scissor assembly coupled to the torsion spring via a drive wheel, the scissor assembly comprising a first link coupled to the trocar link at a first region and a second link coupled to the trocar link at a second region, wherein the torsion spring, when actuated, is configured to rotate the drive wheel to cause, via the scissor assembly, the first trocar, the infusion cannula, the second trocar, and the sensor electrode to be inserted into the user's skin.
10. The device of clause 9 or of any of the preceding clauses, wherein the drive wheel is disposed within a housing and the drive wheel comprises a pin received within a cam slot of the first link, such that rotation of the drive wheel causes the pin to slide within the cam slot and causes the first link to rotate relative to the drive wheel housing.
11. The device of clause 10 or of any of the preceding clauses, wherein the second link is coupled to the first link such that rotation of the first link causes opposite rotation of the second link.
12. The device of any one of clauses 9-11 or of any of the preceding clauses, wherein the drive assembly, when actuated, moves the scissor assembly from an unfired position in which the infusion cannula and sensor electrode are disposed within a housing of the device to an inserted position in which the infusion cannula and sensor electrode extend beyond the housing of the device.
13. The device of clause 12 or of any of the preceding clauses, wherein, in the unfired position, the first link and second link assume an expanded state in which they extend along non-parallel axes, and wherein, in the inserted position, the first link and the second link assume a collapsed state in which they extend parallel to one another.
14. The device of any one of clauses 1-8 or of any of the preceding clauses, wherein the drive assembly further comprises a first drive wheel coupled to the torsion spring and a second drive wheel mated with the first drive wheel such that rotation of the first drive wheel causes rotation of the second drive wheel.
15. The device of clause 14 or of any of the preceding clauses, wherein the first drive wheel comprises a first pin and the second drive wheel comprises a second pin, each of the first pin and the second pin extending into a cam slot of the trocar link such that rotation of the first drive wheel and the second drive wheel causes the trocar link to move axially.
16. The device of clause 15 or of any of the preceding clauses, wherein axial movement of the trocar link in a downward direction causes axial movement of the carrier assembly in the downward direction.
17. The device of any one of clauses 14-16 or of any of the preceding clauses,
   wherein rotation of the first drive wheel in a first direction causes rotation of the second drive wheel in a second, opposite direction.
18. The device of any one of clauses 15-17 or of any of the preceding clauses,
   wherein the cannula carrier comprises a first cam track configured to releasably receive the first pin therein and a second cam track configured to releasably receive the second pin therein.
19. The device of clause 18 or of any of the preceding clauses, wherein the first and second pins engage the first and second cam tracks, respectively, when the trocar assembly and the carrier assembly are each in a downwardly inserted position, and wherein the first and second pins disengage from the first and second cam tracks when the trocar assembly is retracted upward with respect to the carrier assembly.
20. An integrated sensor and infusion device comprising:
   a torsion spring;
   a scissor assembly coupled to the torsion spring, the scissor assembly comprising a first link and a second link;
   a slide having first and second trocars coupled thereto, the slide coupled to the first link at a first region and coupled to the second link at a second region;
   an infusion cannula removably coupled to the first trocar; and
   a sensor electrode removably coupled to the second trocar,
   wherein the torsion spring, when actuated, is configured to cause, via the scissor assembly, the slide to move axially to drive the first trocar, the infusion cannula, the second trocar, and the sensor electrode into a user's skin.
21. The device of clause 20, wherein each of the components of the device are configured to be housed within a device housing prior to insertion of the first trocar, the infusion cannula, the second trocar, and the sensor electrode into the user's skin, and wherein the first trocar, the infusion cannula, the second trocar, and the sensor electrode extend out of the housing for insertion into the user's skin.
22. The device of any one of clauses 20-21, wherein a first amount of rotation by the torsion spring drives the first trocar, the infusion cannula, the second trocar, and the sensor electrode into the user's skin, and wherein a second amount of rotation retracts the first trocar and the second trocar from the user's skin while leaving the infusion cannula and the sensor electrode in the user's skin.
23. The device of clause 22 or of any of the clauses 20-22, wherein the first amount of rotation is approximately 120 degrees, and the second amount of rotation an additional approximately 50 degrees.
24. The device of any one of clauses 20-23, wherein the torsion spring is actuated via a manual trigger mechanism.
25. The device of any one of clauses 20-23 or of any of the clauses 20-24, wherein the torsion spring is actuated via a remote trigger mechanism.
26. The device of any one of clauses 20-24 or of or of any of the clauses 20-25,
   wherein the first trocar is configured to extend within a lumen of the infusion cannula.
27. The device of any one of clauses 20-26, wherein the sensor electrode is configured to be removably received within a recess of the second trocar.
28. The device of any one of clauses 20-27, wherein the scissor assembly is coupled to the torsion spring via a drive wheel disposed within a drive wheel housing and the drive wheel comprises a pin received within a cam slot of the first link, such that rotation of the drive wheel causes the pin to slide within the cam slot and causes the first link to rotate relative to the drive wheel housing.
29. The device of clause 28 or of any of the clauses 20-28, wherein the second link comprises a pin extending into the cam slot of the first link such that rotation of the first link causes the pin to slide within the cam slot.
30. The device of any one of clauses 20-29, wherein the drive assembly, when actuated, moves the scissor assembly from an unfired position in which the infusion cannula and sensor electrode are disposed within a housing of the device to an inserted position in which the infusion cannula and sensor electrode extend beyond the housing of the device.
31. The device of clause 30 or of any of the clauses 20-30, wherein, in the unfired position, the first link and second link assume an expanded state in which they extend along non-parallel axes, and wherein, in the inserted position, the first link and the second link assume a collapsed state in which they extend parallel to one another.
32. An integrated sensor and infusion device comprising:
   a torsion spring;
   a first drive wheel coupled to the torsion spring;
   a second drive wheel mated with the first drive wheel such that rotation of the first drive wheel causes rotation of the second drive wheel;
   a slide having first and second trocars coupled thereto, the slide coupled to the first drive wheel at a first region and coupled to the second drive wheel at a second region;
   an infusion cannula removably coupled to the first trocar; and
   a sensor electrode removably coupled to the second trocar,
   wherein the torsion spring, when actuated, is configured to rotate the first drive wheel to cause the slide to move axially to drive the first trocar, the infusion cannula, the second trocar, and the sensor electrode into a user's skin.
33. The device of clause 32, wherein each of the components of the device are configured to be housed within a device housing prior to insertion of the first trocar, the infusion cannula, the second trocar, and the sensor electrode into the user's skin, and wherein the first trocar, the infusion cannula, the second trocar, and the sensor electrode extend out of the housing for insertion into the user's skin.
34. The device of any one of clauses 32-33, wherein a first amount of rotation by the torsion spring drives the first trocar, the infusion cannula, the second trocar, and the sensor electrode into the user's skin, and wherein a second amount of rotation retracts the first trocar and the second trocar from the user's skin while leaving the infusion cannula and the sensor electrode in the user's skin.
35. The device of clause 34 or of any of the clauses 32-34, wherein the first amount of rotation is approximately 180 degrees, and the second amount of rotation is an additional approximately 180 degrees.
36. The device of any one of clauses 32-35, wherein the torsion spring is actuated via a manual trigger mechanism.
37. The device of any one of clauses 32-35 or of any of the clauses 32-36, wherein the torsion spring is actuated via a remote trigger mechanism.
38. The device of any one of clauses 32-37, wherein the first trocar is configured to extend within a lumen of the infusion cannula.
39. The device of any one of clauses 32-38, wherein the sensor electrode is configured to be removably received within a recess of the second trocar.
40. The device of any one of clauses 32-39, wherein the first drive wheel comprises a first pin and the second drive wheel comprises a second pin, each of the first pin and the second pin extending into a cam slot of the slide such that rotation of the first drive wheel and the second drive wheel causes the slide to move axially.
41. The device of clause 40 or of any of the clauses 32-40, wherein axial movement of the slide in a downward direction causes axial movement of the carrier assembly in the downward direction.
42. The device of any one of clauses 32-41 wherein rotation of the first drive wheel in a first direction causes rotation of the second drive wheel in a second, opposite direction.
43. The device of any one of clauses 32-42, further comprising a cannula carrier including a first cam track configured to releasably receive the first pin therein and a second cam track configured to releasably receive the second pin therein.
44. The device of clause 43 or of any of the clauses 32-43, wherein the first and second pins engage the first and second cam tracks, respectively, when a trocar assembly and the carrier are each in a downwardly inserted position, and wherein the first and second pins disengage from the first and second cam tracks when the trocar assembly is retracted upward with respect to the carrier assembly.
45. The device of any one of the preceding clauses, wherein the reservoir contains insulin.
46. The device of any one of the preceding clauses, wherein the sensor electrode comprises a glucose monitor electrode.
47. The device of any one of the preceding clauses, wherein, in the inserted state, the infusion cannula extends at least 5 mm into the user's skin.
48. The device of any one of the preceding clauses, wherein, in the inserted state, the sensor electrode extends at least 8 mm into the user's skin.
49. The device of any one of the preceding clauses, wherein the infusion cannula and the sensor electrode are laterally spaced apart from one another by at least 10 mm.
50. The device of any one of the preceding clauses, wherein, when positioned over the user's skin, a greatest height of the device over the user's skin is less than about 20 mm.
51. A method for operating an integrated sensor and infusion device, the method comprising:
   disposing a device housing over a user's skin, the device comprising:
      a drive assembly comprising a torsion spring;
      an infusion cannula fluidically coupled to a reservoir configured to hold medicament therein; and
      a sensor electrode;
   actuating the drive assembly to rotate the torsion spring, thereby driving the infusion cannula and the sensor electrode into a user's skin;
   obtaining physiological measurement(s) via the sensor electrode; and
   based at least in part on the physiological measurement(s), delivering medicament into the user's body via the infusion cannula.
52. The method of clause 51, wherein the medicament comprises insulin.
53. The method of any one of clauses 51-52, wherein the physiological measurement(s) comprises a blood glucose measurement.
54. The method of any one of clauses 51-53, wherein the device further comprises a cannula carrier coupled to the infusion cannula and the sensor electrode.
55. The method of any one of clauses 51-54, wherein the device further comprises a trocar assembly comprising a trocar link coupled to a first trocar configured to releasably engage the infusion cannula and a second trocar configured to releasably engage the sensor electrode.
56. The method of any one of clauses 51-55, wherein the drive assembly further comprises a scissor assembly coupled to the torsion spring, and wherein rotation of the torsion spring causes the scissor assembly to move from an expanded configuration in which first and second links of the scissor assembly are oriented along intersecting axes to a collapsed configuration in which the first and second links of the scissor assembly are oriented substantially parallel to one another.
57. The method of any one of clauses 51-55 or of any of the clauses 51-56, wherein the drive assembly further comprises a first drive wheel coupled to the torsion spring and a second drive wheel mated with the first drive wheel such that rotation of the first drive wheel causes rotation of the second drive wheel, and wherein rotation of the first drive wheel and the second drive wheel drives the infusion cannula and the sensor electrode into a user's skin.
58. An integrated sensor and infusion device, the device comprising:
   insertion means comprising:
   a first carrier means for carrying an infusion means and a sensing means;
   a second carrier means for carrying a first insertion means configured to couple to the infusion means and a second insertion means configured to couple to the sensing means; and
   a drive means configured to drive the second carrier means and the first carrier means axially downward to insert the infusion means and the sensing means into a user's skin.
59. The device of clause 58, wherein the drive means is further configured to retract the second carrier means axially upward while leaving the first carrier means in the axially downward position with the infusion means and sensing means inserted into the user's skin.
60. The device of any one of clauses 58-59, wherein the infusion means comprises a cannula fluidically coupled to a reservoir.
61. The device of any one of clauses 58-60, wherein the sensing means comprises a sensor electrode.
62. The device of any one of clauses 58-61, wherein the first insertion means comprises a first trocar.
63. The device of any one of clauses 58-62, wherein the second insertion means comprises a second trocar.
64. The device of any one of clauses 58-63, wherein the drive means comprises a torsion spring.
65. The device of clause 64 or of any of the clauses 58-64, wherein the drive means comprises a scissor assembly coupled to the torsion spring.
66. The device of clause 64 or of any of the clauses 58-65, wherein the drive means comprises a dual-crank assembly coupled to the torsion spring.

## Claims

1. An integrated sensor and infusion device, the device comprising:
a reservoir assembly comprising a reservoir configured to retain medicament therein;
a sensor electronics assembly configured to receive signals from a sensor; and
an insertion assembly comprising:
a carrier assembly comprising a cannula carrier, an infusion cannula extending downwardly away from the cannula carrier, and a sensor electrode extending downwardly away from the cannula carrier at a position laterally spaced apart from the infusion cannula, wherein the infusion cannula is fluidically coupled to the reservoir;
a trocar assembly comprising a trocar link, a first trocar configured to removably engage the infusion cannula, and a second trocar configured to removably engage the sensor electrode, the trocar assembly removably coupled to the carrier assembly; and
a drive assembly comprising a torsion spring coupled to the trocar assembly such that, when actuated, the torsion spring rotates to drive the trocar assembly and the carrier assembly axially downward to insert the first trocar, the infusion cannula, the second trocar, and the sensor electrode into a user's skin.

2. The device of claim 1, wherein each of the components of the device are configured to be housed within a device housing prior to insertion of the first trocar, the infusion cannula, the second trocar, and the sensor electrode into the user's skin, and wherein the first trocar, the infusion cannula, the second trocar, and the sensor electrode extend out of the housing for insertion into the user's skin.

3. The device of any one of claims 1-2, wherein a first amount of rotation by the torsion spring drives the first trocar, the infusion cannula, the second trocar, and the sensor electrode into the user's skin, and wherein a second amount of rotation retracts the first trocar and the second trocar from the user's skin while leaving the infusion cannula and the sensor electrode in the user's skin,
particularly wherein the first amount of rotation is approximately 180 degrees, and the second amount of rotation is an additional approximately 180 degrees.

4. The device of any one of claims 1-3, wherein the torsion spring is actuated via a manual trigger mechanism, and/or
wherein the torsion spring is actuated via a remote trigger mechanism.

5. The device of any one of claims 1-4, wherein the first trocar is configured to extend within a lumen of the infusion cannula, and/or
wherein the sensor electrode is configured to be removably received within a recess of the second trocar.

6. The device of any one of claims 1-5, wherein the drive assembly further comprises a scissor assembly coupled to the torsion spring via a drive wheel, the scissor assembly comprising a first link coupled to the trocar link at a first region and a second link coupled to the trocar link at a second region, wherein the torsion spring, when actuated, is configured to rotate the drive wheel to cause, via the scissor assembly, the first trocar, the infusion cannula, the second trocar, and the sensor electrode to be inserted into the user's skin.

7. The device of claim 6, wherein the drive wheel is disposed within a housing and the drive wheel comprises a pin received within a cam slot of the first link, such that rotation of the drive wheel causes the pin to slide within the cam slot and causes the first link to rotate relative to the drive wheel housing, particularly wherein the second link is coupled to the first link such that rotation of the first link causes opposite rotation of the second link,
and/or wherein the drive assembly, when actuated, moves the scissor assembly from an unfired position in which the infusion cannula and sensor electrode are disposed within a housing of the device to an inserted position in which the infusion cannula and sensor electrode extend beyond the housing of the device, particularly wherein, in the unfired position, the first link and second link assume an expanded state in which they extend along non-parallel axes, and wherein, in the inserted position, the first link and the second link assume a collapsed state in which they extend parallel to one another.

8. The device of any one of claims 1-7, wherein the drive assembly further comprises a first drive wheel coupled to the torsion spring and a second drive wheel mated with the first drive wheel such that rotation of the first drive wheel causes rotation of the second drive wheel.

9. The device of claim 8, wherein the first drive wheel comprises a first pin and the second drive wheel comprises a second pin, each of the first pin and the second pin extending into a cam slot of the trocar link such that rotation of the first drive wheel and the second drive wheel causes the trocar link to move axially, particularly wherein axial movement of the trocar link in a downward direction causes axial movement of the carrier assembly in the downward direction,
and/or wherein rotation of the first drive wheel in a first direction causes rotation of the second drive wheel in a second, opposite direction.

10. The device of any one of claim 9, wherein the cannula carrier comprises a first cam track configured to releasably receive the first pin therein and a second cam track configured to releasably receive the second pin therein,
particularly wherein the first and second pins engage the first and second cam tracks, respectively, when the trocar assembly and the carrier assembly are each in a downwardly inserted position, and wherein the first and second pins disengage from the first and second cam tracks when the trocar assembly is retracted upward with respect to the carrier assembly.

11. An integrated sensor and infusion device comprising:
a torsion spring;
a scissor assembly coupled to the torsion spring, the scissor assembly comprising a first link and a second link;
a slide having first and second trocars coupled thereto, the slide coupled to the first link at a first region and coupled to the second link at a second region;
an infusion cannula removably coupled to the first trocar; and
a sensor electrode removably coupled to the second trocar,
wherein the torsion spring, when actuated, is configured to cause, via the scissor assembly, the slide to move axially to drive the first trocar, the infusion cannula, the second trocar, and the sensor electrode into a user's skin.

12. An integrated sensor and infusion device comprising:
a torsion spring;
a first drive wheel coupled to the torsion spring;
a second drive wheel mated with the first drive wheel such that rotation of the first drive wheel causes rotation of the second drive wheel;
a slide having first and second trocars coupled thereto, the slide coupled to the first drive wheel at a first region and coupled to the second drive wheel at a second region;
an infusion cannula removably coupled to the first trocar; and
a sensor electrode removably coupled to the second trocar,
wherein the torsion spring, when actuated, is configured to rotate the first drive wheel to cause the slide to move axially to drive the first trocar, the infusion cannula, the second trocar, and the sensor electrode into a user's skin.

13. The device of any one of the preceding claims, wherein the reservoir contains insulin, and/or
wherein the sensor electrode comprises a glucose monitor electrode, and/or
wherein, in the inserted state, the infusion cannula extends at least 5 mm into the user's skin, and/or
wherein, in the inserted state, the sensor electrode extends at least 8 mm into the user's skin, and/or
wherein the infusion cannula and the sensor electrode are laterally spaced apart from one another by at least 10 mm, and/or
wherein, when positioned over the user's skin, a greatest height of the device over the user's skin is less than about 20 mm.

14. A method for operating an integrated sensor and infusion device, the method comprising:
disposing a device housing over a user's skin, the device comprising:
a drive assembly comprising a torsion spring;
an infusion cannula fluidically coupled to a reservoir configured to hold medicament therein; and
a sensor electrode;
actuating the drive assembly to rotate the torsion spring, thereby driving the infusion cannula and the sensor electrode into a user's skin;
obtaining physiological measurement(s) via the sensor electrode; and
based at least in part on the physiological measurement(s), delivering medicament into the user's body via the infusion cannula.

15. An integrated sensor and infusion device, the device comprising: insertion means comprising:
a first carrier means for carrying an infusion means and a sensing means;
a second carrier means for carrying a first insertion means configured to couple to the infusion means and a second insertion means configured to couple to the sensing means; and
a drive means configured to drive the second carrier means and the first carrier means axially downward to insert the infusion means and the sensing means into a user's skin.
